(19) **Europäisches Patentamt**

**European Patent Office**

**Office européen des brevets**

(11) **EP 1 388 801 A2**

(12) **EUROPEAN PATENT APPLICATION**

(43) Date of publication:
**11.02.2004 Bulletin 2004/07**

(51) Int Cl.7: **G06F 19/00**

(21) Application number: **03254842.2**

(22) Date of filing: **01.08.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **08.08.2002 US 402566 P**
**31.03.2003 US 403762**

(71) Applicant: **Agilent Technologies, Inc.**
**Palo Alto, CA 94306 (US)**

(72) Inventors:
• **Kincaid, Robert**
 **Half Moon Bay, California 94019 (US)**
• **Vailaya, Aditya**
 **Santa Clara, California 95054 (US)**

(74) Representative: **Exell, Jonathan Mark et al**
**Williams Powell**
**Morley House**
**26-30 Holborn Viaduct**
**London EC1A 2BP (GB)**

(54) **Methods and system for simultaneous visualization and manipulation of multiple data types**

(57) Software systems and methods for organizing and manipulating diverse data sets to facilitate identification, trends, correlations and other useful relationships among the data. Extremely large data set such as microarray data and other biological data are graphically displayed and sorted in an effort to develop visual similarities, correlations or trends that can be seen by a user of the present invention. Various schemes for graphical representations of the data, as well as sorting schemes are provided. Additionally, non-experimental or other data can be displayed and tracked along with the data upon which the sorting schemes are processed.

```
┌─────────────────┐
│ Load experimental│
│ data into an n x m│──── S1
│ matrix          │
└─────────────────┘
        │
        ▼
┌─────────────────┐
│ Load non-experimental│
│ normalized data into │──── S3
│ an n x y matrix │
└─────────────────┘
        │
        ▼
┌─────────────────┐
│ Link "n-value" of│
│ non-experimental data│──── S5
│ with "n-value" of│
│ experimental data│
└─────────────────┘
        │
        ▼
┌─────────────────┐
│ Load non-experimental│
│ normalized data into │──── S7
│ a z x m matrix  │
└─────────────────┘
        │
        ▼
┌─────────────────┐
│ Link "m-value" of│
│ non-experimental data│──── S9
│ with "m-value" of│
│ experimental data│
└─────────────────┘
        │
        ▼
┌─────────────────────────┐
│ Display experimental data│
│ as graphical display and │──── S11
│ display non-experimental data│
│ adjacent the graphical display matrix│
└─────────────────────────┘
        │
        ▼
     ( S13 )
```

FIG. 7A

**Description**

**CROSS-REFERENCE**

This application claims the benefit of U.S. Provisional Application No. 60/402,566, filed August 8, 2002, which application is incorporated herein, in its entirety, by reference thereto.

**FIELD OF THE INVENTION**

The present invention pertains to software systems and methods for organizing and manipulating diverse data sets to facilitate identification, trends, correlations and other useful relationships among the data.

**BACKGROUND OF THE INVENTION**

The advent of new experimental technologies that support molecular biology research have resulted in an explosion of data and a rapidly increasing diversity of biological measurement data types. Examples of such biological measurement types include gene expression from DNA microarray or Taqman experiments, protein identification from mass spectrometry or gel electrophoresis, cell localization information from flow cytometry, phenotype information from clinical data or knockout experiments, genotype information from association studies and DNA microarray experiments, etc. This data is rapidly changing. New technologies frequently generate new types of data.

Understanding observed trends in gene or protein expression often require correlating this data with additional information such as phenotype information, clinical patient data, putative drug treatments dosages, etc. Even when fairly rigorous computational techniques such as machine learning-based clustering or classification schemes are used, the results of these techniques are typically cross-checked with observed phenotypes or clinical diagnoses to interpret what the computational results might mean.

Currently, correlations of the experimental data with types of additional information as exemplified above are done by manually (i.e., visually) inspecting the additional (e.g., clinical) data and visually comparing it with the experimental data to look for similarities (i.e., correlations) between experimental and observed phenomena. For example, a researcher might notice a highly up or down regulated gene during inspection of a microarray experiment and then explore the available clinical data to see if any observed clinical data correlates with the known function of the gene involved in the microarray experiment. Finding correlations in this manner could be described as a "hit-or-miss" procedure and is also dependent upon the accumulated knowledge of the researcher. Further, the large volumes of data that are generated by current experimental data generating procedures, such as microarray procedures, for example, makes this method of correlating an extremely tedious, if not impossible task.

Efforts at consolidating the data to be analyzed for correlations between experimental results and observed phenomena have been made by attempting to consolidate all the data to be viewed into massive spreadsheets or tabular displays. However, the usefulness of these types of approaches has been limited because, due to the sheer volumes of data that usually need to be analyzed, it becomes impossible to view all relevant experimental data together, at once, on a single screen to allow visual comparison. Accordingly, it becomes necessary to provide split views, scrolling or multiple windows in order to view all of the data needed for performing the analysis. Not only does this make it difficult to easily make visual comparisons among the data contained in different screens, windows or views, but the ability to manipulate the data so as to make visual comparisons according to different characterizations of the data (different types of sorting, clustering, classification, etc.) to search for trends, correlations or other insights, becomes unwieldy and problematic.

Efforts have been made in attempting to visualize and discover overall gene expression patterns from large gene expression data sets with little success. For example, scatter plots and parallel coordinate techniques available with Spotfire 4.0 and Spotfire 5.0 were used by Pan in an attempt to identify expressed sequence tags (ESTs) having expression patterns similar to those of known genes. Both the expression patterns of the ESTs as well as those of the known genes were obtained from a data set including melanoma samples and normal (control) samples provided by National Human Genome Research Institute (see Pan, Zhijian: "Application Project: Visualized Pattern Matching of Malignant Melanoma with Spotfire and Table Lens", http//:www.cs.umd.edu/class/spring2001/cmsc838b/Apps /presentations/Zhijian_Pan/. The use of scatter plots was reported to be incapable of managing the complexity of the data set being examined. The use of parallel coordinates with Spotfire 5.0 was more promising, in that it was capable of displaying all thirty-eight experimental conditions on a single page, where similarities in expression patterns could be searched for.

Table Lens was also employed by the same researcher to visualize expression patterns of the ESTs and known genes. However, it was reported that Table Lens was ineffective, and "very difficult" for use in finding matching patterns. Neither Spotfire (4.0 or 5.0) was used to compare expression or other experimental data with supporting clinical data or data sets of any other type, but were only used in attempting to group like data within the experimental data set.

More powerful methods of combining widely diverse, but related and potentially correlated biological data sets are needed to improve the ease, speed and efficiency of correlating information in these data sets. Further, more powerful methods are needed to improve the probability that such correlations will be identified.

According to one aspect of the present invention there is provided a method according to claim 1.

According to another aspect of the present invention there is provided a system according to claim 14.

The present invention provides systems and methods for manipulating large data sets for visually identifying relationships among the data that can be useful to a researcher that cannot be achieved mentally or by using normal computational methods or systems. By manipulating the data according to the present methods, most, if not all relevant data can be inspected simultaneously in graphical form. Data can be easily and quickly manipulated by sorting or re-ordering both rows and columns to expose potentially meaningful correlations and trends in the data which are easily observed.

Data may be presented in a way that all of an underlying matrix can be generally displayed, while a more detailed view of a selected region of the data can be simultaneously viewed and manipulated. Numerical data or measurements may be combined with classification or other descriptive or non-numerical data, which is then tracked with the present system to maintain proper correlation with the numerical data as the numerical data is sorted and manipulated. A very intuitive user interface for combining different data types into a single view is presented.

A variety of different techniques for graphically representing the data are also disclosed, as well as various sorting and sub-sorting techniques. Additionally, docking features are provided for combining predefined matrices of similar or disparate data.

The present invention provides extremely powerful techniques for visualizing the massive datasets generated by high-throughput experiments such as DNA microarrays. Further, the results of these experiments can be visually manipulated to look for trends and correlations using simple analysis techniques or more sophisticated analytical tools such as clustering or classificication algorithms. Calculated data can even be incorporated into the dataset being examined by the invention.

Current algorithmic techniques are quite powerful, but usually directed toward looking at specific pre-defined correlations or trends. The present invention allows approaching the data with no particular predisposition and can be used to provide insight as to which computational techniques might be useful.

Examples of the present invention will now be described in detail with reference to the accompanying drawings in which:

Fig. 1 shows an example of a portion of a conventional heat map visualization 200 that is currently available to users.

Fig. 2 shows a screen display resultant from using a visualization system described in co-pending and commonly owned Application Serial No. 10/209,477 filed July 30, 2002 and titled "Method of Identifying Trends, Correlations, and Similarities Among Diverse Biological Data Sets and System for Facilitating Identification".

Fig. 3 shows a screen display after sorting the data displayed in Fig. 2.

Fig. 4 shows a screen display 100 resultant from using a visualization system according to the present invention.

Fig. 5 shows a screen display resulting from performing a column sort on the data shown in Fig. 4, according to the present invention.

Fig. 6 shows the display order resulting after a row sort was performed subsequent to column sort described with regard to Fig. 5.

Figs. 7A-7B show a flow chart which outlines basic procedures for preparing and displaying a visualization using the system according to the present invention, and for the manipulations of the data displayed.

Fig 8A shows a simple 3 x 4 matrix referred to for purposes of demonstrating concepts of similarity sorting according to the present invention.

Fig. 8B shows a popup menu that may be invoked by the user to perform sorting manipulations and/or access additional annotation data.

Fig. 8C shows the matrix of Fig. 8A, after selection of row 202 for performance of a similarity sort based thereon according to the present invention.

Fig. 8D shows the resulting order of the cells of the matrix after performing a similarity sort based upon the selection shown in Fig. 8C.

Fig. 9 shows the results of a similarity row sort according to the present invention, wherein the sort was based upon the row identified as gene "DUSP1".

Fig. 10 shows a visualization that employs an alternative representation of the traditional heat map view in the experimental data portion of the matrix according to the present invention.

Fig. 11 shows a visualization that employs another alternative representation of the traditional heat map view in the experimental data portion of the matrix according to the present invention.

Fig. 12 shows a visualization that employs still another alternative representation of the traditional heat map view in the experimental data portion of the matrix according to the present invention.

Fig. 13 shows a highly compressed visualization for maximizing the number of rows of experimental data that can be individually visualized in the matrix on a single screen.

Fig. 14 shows a visualization of a pop-up display that may be accessed to display annotations that are pertinent to a cell selected by the user.

Fig. 15 shows a modified visualization according to the present invention which provides a generalized view of all of the experimental data in a compressed experimental data matrix, while at the same time providing an non-compressed view of a selected portion of the experimental data in a matrix.

Figs. 16-18 illustrate user interface mechanism functions provided for combining related data of different types into a single unified visualization by the system according to the present invention.

## DETAILED DESCRIPTION OF THE INVENTION

Before the present methods, tools and system are described, it is to be understood that this invention is not limited to particular data sets, manipulations, tools or steps described, as such may, of course, vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a", "and", and "the" include plural referents unless the context clearly dictates otherwise. Thus, for example, reference to "a gene" includes a plurality of such genes and reference to "the array" includes reference to one or more arrays and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

## DEFINITIONS

The term "cell", when used in the context describing a data table or heat map, refers to the data value at the intersection of a row and column in a spreadsheet-like data structure or heat map; typically a property/value pair for an entity in the spreadsheet, e.g. the expression level for a gene.

"Color coding" refers to a software technique which maps a numerical or categorical value to a color value, for example representing high levels of gene expression as a reddish color and low levels of gene expression as greenish colors, with varying shade/intensities of these colors representing varying degrees of expression. Color-coding is not limited in application to expression levels, but can be used to differentiate any data that can be quantified, so as to distinguish relatively high quantity values from relatively low quantity values. Additionally, a third color can be employed for relatively neutral or median values, and shading can be employed to provide a more continuous spectrum of the color indicators.

The term "data mining" refers to a computational process of extracting higher-level knowledge from patterns of data in a database. Data mining is also sometimes referred to as "knowledge discovery".

The term "down-regulation" is used in the context of gene expression, and refers to a decrease in the amount of messenger RNA (mRNA) formed by expression of a gene, with respect to a control.

"Gel electrophoresis" refers to a biological technique for separating and measuring amounts of protein fragments in a sample. Migration of a protein fragment across a gel is proportional to its mass and charge. Different fragments of proteins, prepared with stains, will accumulate on different segments of the gel. Relative abundance of the protein fragment is proportional to the intensity of the stain at its location on the gel.

The term "gene" refers to a unit of hereditary information, which is a portion of DNA containing information required to determine a protein's amino acid sequence.

"Gene expression" refers to the level to which a gene is transcribed to form messenger RNA molecules, prior to protein synthesis.

"Gene expression ratio" is a relative measurement of gene expression, wherein the expression level of a test sample is compared to the expression level of a reference sample.

A "gene product" is a biological entity that can be formed from a gene, e.g. a messenger RNA or a protein.

A "heat map" or "heat map visualization" is a visual representation of a tabular data structure of gene expression values, wherein color-codings are used for displaying numerical values. The numerical value for each cell in the data table is encoded into a color for the cell. Color encodings run on a continuum from one color through another, e. g. green to red or yellow to blue for gene expression values. The resultant color matrix of all rows and columns in the data set forms the color map, often referred to as a "heat map" by way of analogy to modeling of thermodynamic data.

A "hypothesis" refers to a provisional theory or assumption set forth to explain some class of phenomenon.

An "item" refers to a data structure that represents a biological entity or other entity. An item is the basic "atomic" unit of information in the software system.

The term "mass spectrometry" refers to a set of techniques for measuring the mass and charge of materials such as protein fragments, for example, such as by gathering data on trajectories of the materials/fragments through a measurement chamber. Mass spectrometry is particularly useful for measuring the composition (and/or relative abundance) of proteins and peptides in a sample.

A "microarray" or "DNA microarray" is a high-throughput hybridization technology that allows biologists to probe the activities of thousands of genes under diverse experimental conditions. Microarrays function by selective binding (hybridization) of probe DNA sequences on a microarray chip to fluorescently-tagged messenger RNA fragments from a biological sample.

The amount of fluorescence detected at a probe position can be an indicator of the relative expression of the gene bound by that probe.

The term "normalize" refers to a technique employed in designing database schemas. When designing efficiently stored relational data, the designer attempts to reduce redundant entries by "normalizing" the data, which may include creating tables containing single instances of data whenever possible. Fields within these tables point to entries in other tables to establish one to one, one to many or many to many relationships between the data. In contrast, the term "de-normalize" refers to the opposite of normalization as used in designing database schemas. De-normalizing means to flatten out the space efficient relational structure resultant from normalization, often for the purposes of high speed access that avoid having to follow the relationship links between tables.

The term "promote" refers to an increase of the effects of a biological agent or a biological process.

A "protein" is a large polymer having one or more sequences of amino acid subunits joined by peptide bonds.

The term "protein abundance" refers to a measure of the amount of protein in a sample; often done as a relative abundance measure vs. a reference sample.

"Protein/DNA interaction" refers to a biological process wherein a protein regulates the expression of a gene, commonly by binding to promoter or inhibitor regions.

"Protein/Protein interaction" refers to a biological process whereby two or more proteins bind together and form complexes.

A "sequence" refers to an ordered set of amino acids forming the backbone of a protein or of the nucleic acids forming the backbone of a gene.

The term "overlay" or "data overlay" refers to a user interface technique for superimposing data from one view upon data in a different view; for example, overlaying gene expression ratios on top of a compressed matrix view.

A "spreadsheet" is an outsize ledger sheet simulated electronically by a computer software application; used frequently to represent tabular data structures.

The term "up-regulation", when used to describe gene expression, refers to an increase in the amount of messenger RNA (mRNA) formed by expression of a gene, with respect to a control.

The term "UniGene" refers to an experimental database system which automatically partitions DNA sequences into a non-redundant sets of gene-oriented clusters. Each UniGene cluster contains sequences that represent a unique gene, as well as related information such as the tissue types in which the gene has been expressed and chromosome location.

The term "view" refers to a graphical presentation of a single visual perspective on a data set.

The term "visualization" or "information visualization" refers to an approach to exploratory data analysis that employs a variety of techniques which utilize human perception; techniques which may include graphical presentation of large amounts of data and facilities for interactively manipulating and exploring the data.

The present invention provides tools and methods for manipulating very large data structures, generally in the form of tabular or spreadsheet type data structures, to organize relevant data for ready visualization by a user attempting to visually identify correlations, trends or other insights among the data. Although the techniques described below use manipulation of heat map visualizations as an example of how the invention can be used, the invention is not limited to heat maps or gene expression data, as any numerical data can be accommodated with the methods and tools described herein.

In addition to providing the data to be analyzed in a readily viewable format, the present invention may also integrate additional data such as annotations, other kinds of experimental data, clinical data, and the like. Using the present techniques, most if not all relevant data can be inspected simultaneously in graphical form. The data can be easily and quickly manipulated by sorting or re-ordering rows and/or columns of the data to expose meaningful correlations and trends in the data which can be easily observed as a result of rearrangement.

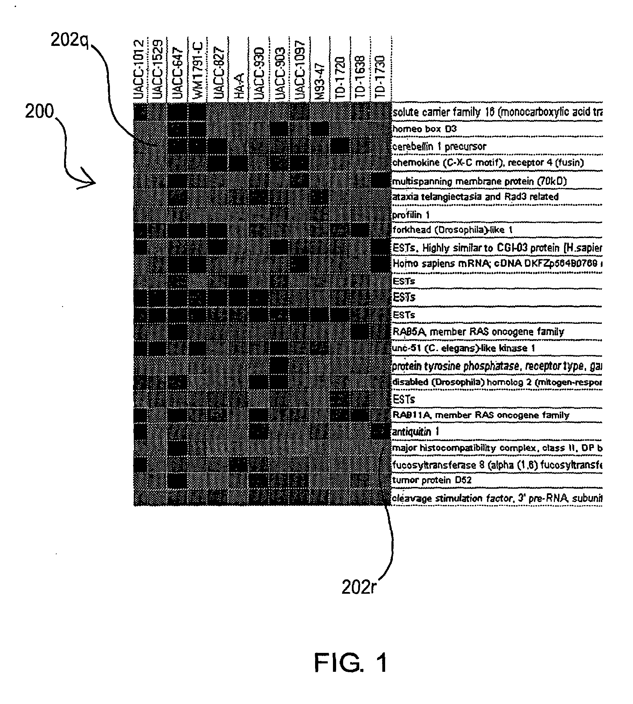

Fig. 1 shows an example of a portion of a conventional heat map visualization 200 that is currently available to users. A standard heat map visualization such as visualization 200 is a static visual representation of a tabular data structure of gene expression values, wherein color-codings are used for displaying numerical values. The numerical value for each cell 202 in the data table is encoded into a color for the cell. Color encodings run on a continuum from one color through another, e.g. green 202g to red 202r or yellow to blue for gene expression values.

Standard heat map visualizations have significant shortcomings as to their usefulness for performing visual correlation analyses. Since these displays are static, the cells in the display 200 cannot be manipulated to form different combinations or views in attempting to find similarities among the experimental data. Although a commonly owned product, known as Synapsia (available from Agilent, Palo Alto, California) provides some limited capability such as simple column sorting or column rearrangement of a heat map, there remains a need for greater manipulation of the data such as provided by the present invention. Further, as noted above, the sheer volumes of data that are generated by current experimental data generating procedures, such as microarray procedures and protein expression measurements, for example, makes it generally impossible to display the contents of all the data that needs to be reviewed on a single display. This further complicates any hope for visually identifying similarities among experiments or gene expression values, since not only is side by side visualization of potentially similar data values not currently possible through use of an automated technique, but the user must additionally switch between screen views to search for similarities, which eliminates the potential for simultaneous viewing of many of the possible combinations of the data.

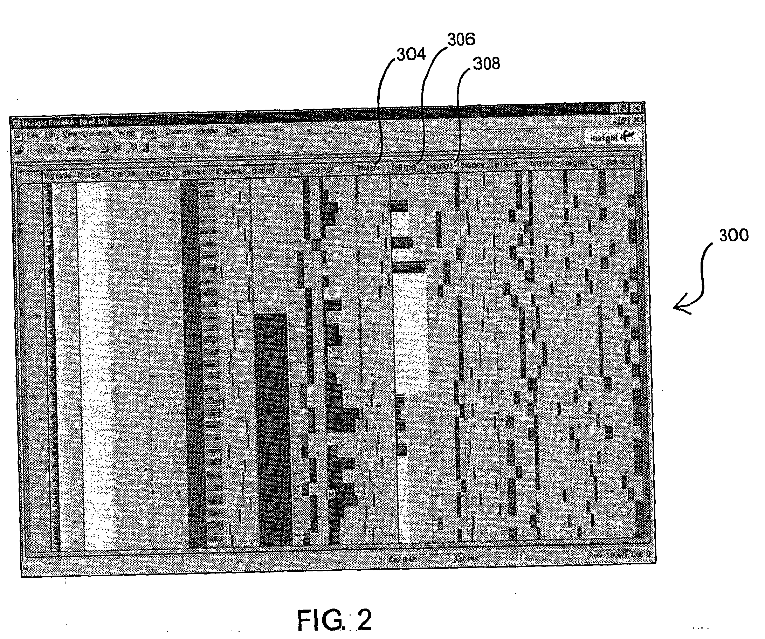

Fig. 2 shows a screen display resultant from using a visualization system described in co-pending and commonly owned Application Serial No. 10/209,477 filed July 30, 2002 and titled "Method of Identifying Trends, Correlations, and Similarities Among Diverse Biological Data Sets and System for Facilitating Identification", which is incorporated herein in its entirety, by reference thereto. The microarray experimental data used to generate the visualization 300 shown was obtained from the National Human Genome Research institute of the National Institutes of Health. Further details regarding the microarray data can be found in Bittner et al., "Molecular classification of cutaneous malignant melanoma by gene expression profiling", Nature, vol. 406, August, 2000, which is incorporated herein, in its entirety, by reference thereto. Experiments were performed with respect to thirty-one subcutaneous melanoma patients using DNA microarrays.

The visualization 300 shows a compressed view of thirty of the thirty-one DNA gene expression microarrays. For each patient, eight thousand and sixty-six individual microarray measurements are displayed in the column labeled log ratio (i.e. the standard log 10 ratio of the signal measurements made for each feature of the array). The underlying table containing the data used to construct the visualization 300 is constructed by de-normalizing (in the database sense) the gene and patient data. Therefore, the expression data column in the underlying table contains 241,980 rows (cells) of gene expression values (i.e., 8,066 x 30). Therefore, each row of the table corresponds to an expression ratio measured on a microarray. Additionally, clinical data as well as patient cluster, and gene specific annotations corresponding to the gene represented by the expression ratios are contained within the respective rows. Since the data set is highly de-normalized, for a given patient, the data in the clinical columns are repeated for each gene measured by that patient's microarray.

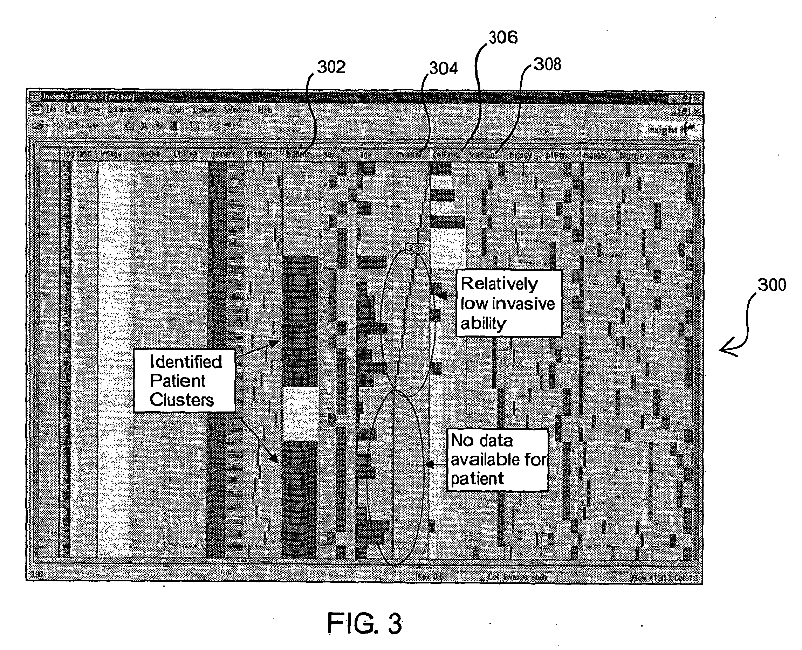

In order to display such a massive number of columns in a single visualization 300 as shown in Fig. 2, the system employs a visualization tool known as Table Lens, which allows the diverse data sets to be compressed, displayed and inspected simultaneously in graphical form on a single display. In the examples shown in Figs. 2 and 3, the system was based on a product known as Eureka, by Inxight. A complete description of the functionality of Table Lens can be found in U.S. Patent Nos. 5,632,009; 5,880,742 and 6,085,202, each of which is incorporated herein, in its entirety, by reference thereto. The resultant visualization 300 is a very dense graphical display representing 241,980 rows of data entirely visible on a single standard computer display. The visualization 300 is highly compressed, with graphical values displayed to represent groups of cell values, since the compression prevents each individual row or cell value from being displayed. For example, the expression values are shown with white indicating the maximum value of the group of expression values represented in the displayable area and the blue indicating the minimum value. This is particularly useful in the log ratio column as there are actually many values represented within a particular "pixel" row due to the high compression of the data to fit within this display. A second important feature is that depending on the sort order chosen for the display; blocks of similar data will appear as colored rectangles. Since some data can be designated as "categories" vs. numerical measurements, this is quite useful. So in this display as you look at the patient id column (the sixth column), it's easy to spot the block of rows corresponding to each patient.

However, in the visualization 300 as shown in Fig. 2, it is difficult to visually detect any really relevant correlations of the data, as no real patterns (other than the patient id blocks) are readily discernible, and no really meaningful sort order has been chosen yet. Fig. 3 shows a rearrangement of the data after sorting first by patient cluster 302 and second by "invasive ability" 304. These two sort criteria were chosen in an effort to verify the assertion in Bittner et al. that the cluster assignment made in that paper based on informative genes does correspond to low invasive ability of the malignancy. As a result of the sorting, a clear correlation can be observed between the two defined patient clusters (in column 302 of the display) and the invasive ability values (shown sorted according to a decreasing order in column 304). Note that the blocks at the bottom portion of the patient id column 302 correspond to the bottom of the invasive ability column 304 having nil values, indicating that this category of patients did not have a recorded invasive ability measurement.

Although the system and methods described with regard to Figs. 2 and 3 can be very useful and powerful in preparing visualizations for the analysis of biological analysis, they also require a significant amount of learning and familiarization with what is otherwise a quite non-intuitive display for those trained in the biological research disciplines. Those users that have not dedicated enough time to fully understand how to manipulate and interpret the display are likely to be confused or intimidated by the graphical representations of the compressed data and as to how to interpret them.

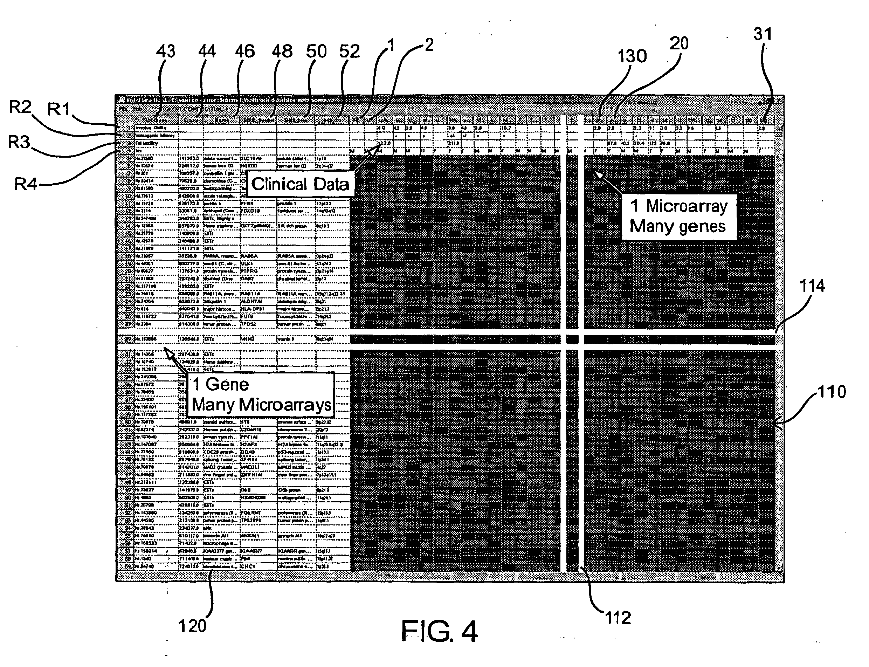

Fig. 4 shows a screen display 100 resultant from using a visualization system according to the present invention, in which the same microarray experimental data used in the visualizations for Figs. 2-3 was used, except that the data associated with all thirty-one DNA gene expression microarrays was loaded into the system of the present invention. The experimental display portion 110 of the visualization 100 is designed to appear as a typical heat map visualization, so that users will be comfortable with viewing and interpreting the data. Unlike a typical heat map visualization, however, the experimental display portion is not a substantially static display, but may be manipulated to gain insight into correlations and similarities among the data displayed, as will be discussed in more detail below. Unlike the display in Figs. 2-3, the experimental data in display portion 110 is not compressed, and therefore not all of the experimental data is shown in Fig. 3, since there will be 8,066 cells of experimental values for each of the arrays 1, 2...31 displayed in the experimental display portion 110. However, using the manipulation techniques described below, the system is designed to reorder the data to group relevant data so that most if not all relevant data can be viewed on a single display 100.

In addition to the experimental data, clinical data and patient data are included in portions 120 and 130 of the visualization 100 adjacent matrix 110 shown in Fig.4. The column 43 labeled "Unigene" contains the Unigene Cluster ID that further identifies the CDNA having been deposited on the microarray, with respect to each of the respective cells in each array 1-31. Thus, for example, Unigene Cluster ID "Hs 23590" is associated with the first row of experimental data 110 as shown in Fig. 4. This identifier is linked to that particular row of array data, so that if the row is reordered within the array, the Unigene Cluster ID is also reordered to the same row that the data assumes, to maintain accuracy of the characterizing clinical data. Likewise, the column of clinical data containing the cloneID (i.e., "Clone") 44 for the CDNA having been deposited on the microarray with respect to each individual microarray reading is linked to the particular row of experimental data that it describes and moves with that row when the row is repositioned. All other columns of clinical data share this characteristic. Columns 46, 48, 50 and 52 contain Name, BNS Symbol, BNS Description, BNS Chr data for each gene having these identification data in its row. The BNS columns 48, 50 and 52 contain information that is all imported from a commonly owned biological naming system, which is described in more detail in co-pending and commonly owned Application Serial No. 10/154,529 filed May 22, 2002 and titled "Biotechnology Information Naming System", which is hereby incorporated in its entirety, by reference thereto. The BNS columns 48, 50 and 52 are only examples of additional descriptive or annotative data that may be displayed along with the experimental data according to the present invention, and the present invention is in no way to be limited to inclusion and use of BNS information in each instance of use of the present invention.

The BNS_Symbol column 48 contains symbols which identify the particular gene in that row that the expression data is being presented for. Examples of such symbols appearing are SLC16A4, HOXd3, ATR, etc. The BNS_Description column 50 contains identifiers which are similar to those in the Name column 46, namely the short descriptive names of the genes. In most cases the BNS_Description column 50 and Name column 46 will contain the same information in respective rows, but since the BNS data is more official and recent, there might be slight differences or updates. The BNS_Chr column 52 identifies the cytogenic chromosome location of the gene in the row in which the information appears. All BNS data is derived from NCBI's LocusLink.

It should be noted that the present invention is not limited to capturing and visualization of the particular types of clinical data identified above, as they are only examples. Any textual or numeric data that can be associated with the experimental data can be added into the visualization.

The visualization 100 normalizes the data displayed which helps to make a more compact set of data to be displayed. Thus, for example, unlike the de-normalized display shown in Figs. 2 and 3, the Unigene Cluster ID "Hs 23590" does not have to be displayed individually for each array included in the display (i.e., thirty-one times, one for each cell in the first row of the experimental data shown), but rather is displayed only once for the row of that experimental data.

Additionally, data such as patient data or clinical data can be included in rows adjacent the experimental data display portion 110. In the example shown in Fig. 4, the first four columns of the display 100 incorporate clinical data and data measured from tissue samples. Row R1 includes invasive ability values for particular arrays of data, which correspond to the de-normalized invasive ability values in the column 304 of Figs. 2 and 3, and row R2 indicates vasculogenic mimicry, where a "+" symbol in a cell of row R2 indicates that the data in the microarray in the column with which that cell is aligned exhibits vasculogenic mimicry and a "-" symbol in a cell indicates that the data in the microarray in the column with which that cell is aligned does not exhibit vasculogenic mimicry. An indication in a cell with the symbols "+/-" indicates that there was a mixed measurement. These symbols and their meanings are also referred to and explained in the Bittner publication referred to above. The data in row R2 corresponds to the de-normalized values represented in column 308 of Figs. 2 and 3. Row R3 includes cell motility values for those arrays that had this measurement taken, and these values correspond to the de-normalized cell motility values displayed in column 306 of Figs. 2 and 3. Row R4 displays the sex of each patient represented by each microarray, where "M" symbolizes male, "F" symbolizes female, and "U" symbolizes that the sex of the patient was not recorded.

Like the additional data in the columns of the display 100 (e.g., columns, 43, 44, 46, 48, 50 and 52) the additional data in the rows which characterize the experimental data (rows R1- R4 in the example of Fig. 4) is also normalized. For example, the indicator "M" displayed in the "Sex" row R4 is indicated only once, but pertains to each of the 8,066 cells in the microarray column 1 with which it is aligned, as compared to the display of Figs. 2-3 which reproduces the indication of "M" or "male" for each of the 8,066 individual values. Due to compression, not all of these values are displayed in Figs. 2-3, as a graphical representation of the compression of the values is substituted for it, but the fact remains, that all of the data is at least in the underlying table from which the display of Figs. 2-3 is generated, and therefore the underlying data must be generated in the underlying table at some point in the process. In the case where the experimental data is microarray data, such data is already normalized within a standard heat map, and so the data must be de-normalized in order to incorporate it into the visualization of Figs. 2-3. This is not the case with the present invention however, so that population of the visualizations according to the present invention is more streamlined.

Likewise, each value in each row of data (clinical data, patient data, etc.) associated with the experimental data display 110 is normalized, in that it is only indicated once, in one cell of the row, and pertains to each experimental data cell underlying that cell (e.g., to all of the data in that microarray column, in the case of the example shown in Fig. 4). Note also that the cells which overlap or intersect the additional rows and columns of non-experimental data (in the upper left hand corner of Fig. 1) are left blank, as they are neither adjacent a row of experimental data nor a column of experimental data. The first column of these cells has been conveniently used to identify the rows of the non-experimental data (rows R1- R4). It should be noted that the present invention is not limited to capturing and visualization of the particular types of clinical data and tissue sample data identified above, as they are only examples. Any textual or numeric data that can be associated with the experimental data can be added into the visualization.

The experimental data 110 can be sorted by column or by row, using the cross-hairs 112, 114. When performing a sort, only the experimental data is considered to determine the sort order, while the non-experimental data follows the repositioning of the rows or columns of data as they are resorted. For example, if a user selects the column highlighted by cross-hair 112 for performing a sort by column, only the rows containing the experimental data (i.e., heat map style visualization display 110 in Fig. 4) are sorted, and the clinical data in rows R1- R4 is locked, since the columns of experimental data that they pertain to do not change their positions in the matrix. Likewise, the clinical data in the columns adjacent the experimental data are not considered for sorting, but are reordered to follow the reordering of the rows of experimental data that results from the sort.

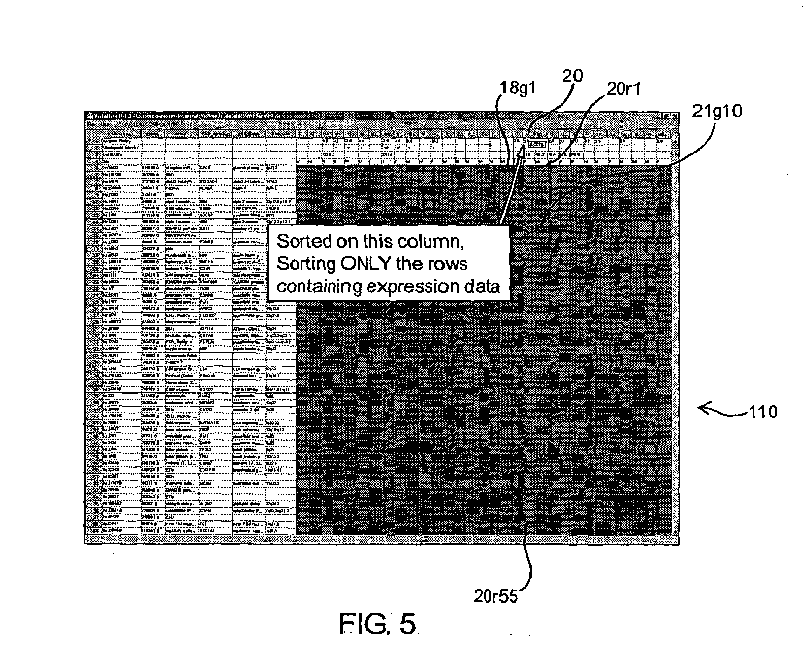

For example, Fig. 5 shows the results of a column sort that was conducted with regard to column 20 of the experimental data. In this example, the cells in column 20 have been sorted according to the cell with the highest degree of up-regulation (which is color-coded red according to the normal heat map visualization schema), with subsequent cells in descending order of expression value down to the lowest value. Of course, the present invention is not to be limited to sorting from highest up-regulated cell, as a reverse sorting order could be performed. Again, because each column has 8,066 cells, not all of the cells are shown in the visualization of Fig. 5. Because the sorting has been performed on the basis of the expression values in column 20, all fifty-three of the cells that are displayed for column 20 are red (20r1 through 20r55). As each of the cells in column 20 are rearranged according to the sort order determined, the entire row of experimental data assumes the same row placement as that of the reordered cell of column 20. Also, the non-experimental data and identification data in the left side of the visualization remains linked with the respective rows that it originally pertained to, and is rearranged according to the sort order of the cells in column 20. In this way, the identifying information/non-experimental data in the cells of columns 42, 44, 46, 48, 50 and 52 remains in the same row relative to the experimental data after re-ordering, thereby maintaining the accuracy of the normalization scheme.

Fig. 5 readily reveals a large concentration of up-regulated expression values, particularly in the upper right portion of the display 110, with some microarray columns having more dissimilar data values than others (see for example, green cells 18g1 and 21g10). However, a general observation that can be made from this sort is that the patients/microarrays on the right side of the matrix 110 appear to have more similarities to microarray/patient column 20 than those on the left side of the matrix 110.

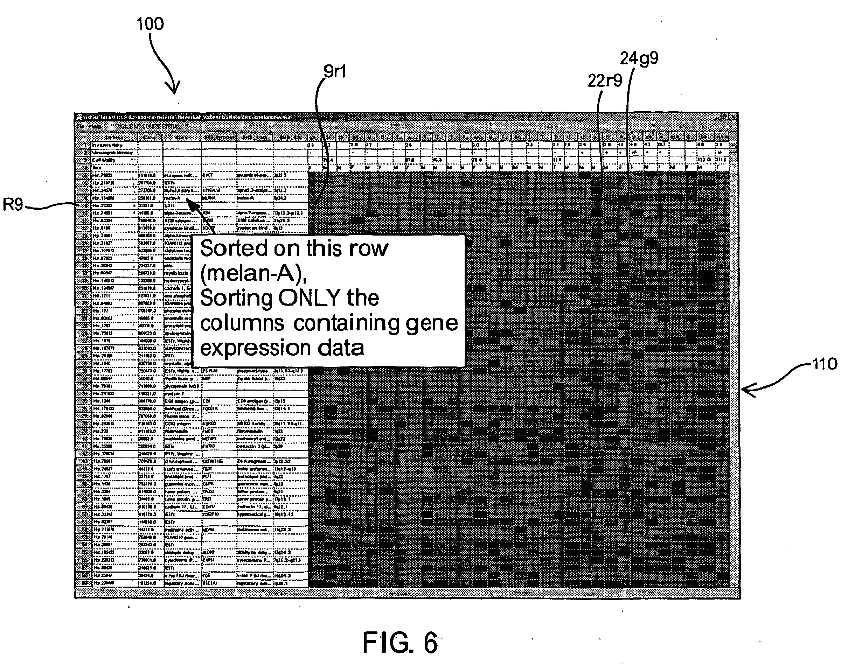

Fig. 6 shows the display order resulting after a row sort that was performed after the column sort described above with regard to Fig. 5. In this example, the sort was performed by outlining the row corresponding to the melan-A gene (row R9) with the cross-hair 114 and selecting a row sort operation. In effect, this row sort operation sorts the cells of row R9 (but only those cells residing within the experimental data portion 110 of the matrix 100), with the left-most cell belonging to the microarray having the highest up-regulation expression value, which, in this case belongs to the microarray that was originally displayed in experimental data column 19 in Figs. 4-5. Accordingly, the array originally placed in experimental data column 19 was reordered or repositioned to assume the position of experimental data column 1 in Fig. 6 and the cell corresponding to the melan-A-gene therefore assumed the first cell position 9r1 in the sorted row. Of course, all of the other corresponding cells in the microarray originally positioned in column 19 are moved to the same respective rows in column 1 so that the entire microarray is represented in column 1. Like the column sort described above, this row sort was performed according to an order displaying the highest up-regulated cell (9r1) first (i.e., the left most cell of the row), with the second cell having the next highest expression level and so forth, down to the lowest expression value in column 31 of row R9. Once again, it is noted that the present invention is not to be limited to sorting from the highest up-regulated cell, as the sort could be based on the lowest expression level, and arranged in an ascending expression level order, for example. When sorting by row according to the data set shown in Fig. 6, the entire results of the sort order of the melan-A-gene can be viewed in row R9, since only 31 microarrays are included in the experimental data. Of course, not all rows are displayed, as indicated above, since this would require some compression scheme, or an extremely large display to represent all 8,066 rows of experimental data.

As noted above, the row sort was performed on the basis of the expression values in row R9 (i.e., Melan-A gene). As each of the cells in row R9 are rearranged according to the sort order determined, the entire column of experimental data assumes the same column placement as that of the reordered cell of row R9. Also, the non-experimental data and identification data in the top portion of the visualization remains linked with the respective columns that it originally pertained to, and is rearranged according to the sort order of the cells in row R9. In this way, the identifying information/ non-experimental data in the cells of rows R1- R4 remains in the same row relative to the experimental data after re-ordering, thereby maintaining the accuracy of the normalization scheme. The non-experimental data on the left side of the visualization 100 remains locked, as it is normalized with respect to the rows of experimental data, which were not reordered in this manipulation.

The results displayed in Fig. 6 show that the user has in effect sorted a group of up-regulated genes (color-coded red in this case) into the upper left comer of the display 110. This sort by melan-A did a fair, but slightly imperfect sorting of the two classes of melanoma patients, as it can be seen that the group on the left side of the display 110 contains a lot of highly up-regulated values, while the group on the right side contains more neutral (e.g., colored coded black or a dark shade of red or green, such as cell 22r9 which is dark red and cell 24g9 which is dark green. Other rows surrounding row R9 in some of the microarrays on the right side also show a large disparity from the concentration of up-regulated cells in the upper left portion of the display 110, owing in part to the previous column sort. For example, column 22 contains a large number of down-regulated or green color-coded cells.

Melan-A is known to play a role in melanoma (hence the name), but if this had been a newly discovered gene, the display in Fig. 6 could have provided an insight to the user as to the potential discovery of a gene which plays an important role in melanoma. Similar to the previous case, the supplementary data in columns 1-7 are essentially locked in place and only the expression data are sorted.

The present invention supports both row and column sorting, as described above, as well as limited column and row re-ordering. This limited column and row re-ordering may be accomplished manually by the user. To accomplish manual reordering, the user can drag-and-drop rows and columns. This is accomplished by simply clicking the column or row header and while holding down the mouse button, dragging it left or right (column) or up or down (row) to its new location.

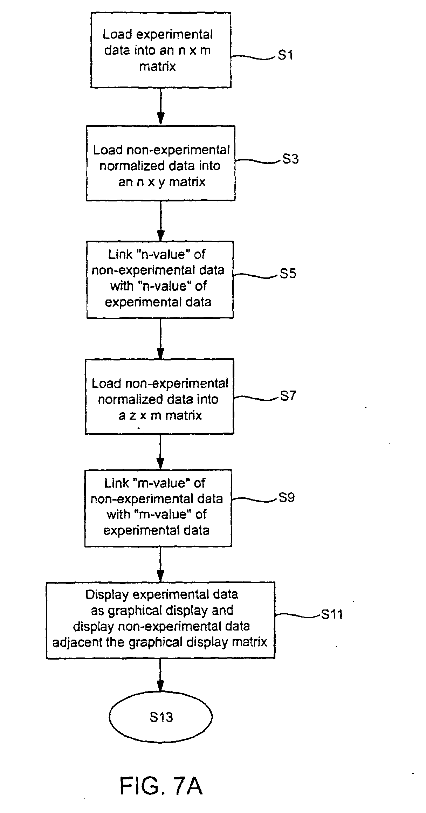

Figs. 7A-7B contain a flow chart which outlines basic procedures for preparing and displaying a visualization 100 using the system according to the present invention, and for the manipulations of the data displayed, such as described above. In step S1, experimental data is inputted into an "n x m" matrix to be displayed as the display portion 110 shown in Figs. 4-6, for example, where "n" is a positive integer representing the number of columns in the matrix, and "m' is a positive integer representing the number of rows in the matrix. Experimental data may be loaded from external sources including, but not limited to, DNA microarray experimental results, relative protein abundance measures derived from mass spectrometry and protein fragment data derived from gel electrophoresis experiments. Experimental data may be loaded as a tab-delimited text file, although the present invention is not limited to this format for loading the data. All data that is seen in the display may be loaded from such a single flat file (tab-delimited text file). Additional lines in the file specify the source experimental data type (e.g., for gene expression values this would be ratio or log-ratio), as well as the position in the full table where the first experimental data representation is to appear (i.e. the row and column). For example, the flat file and system may assume that all experimental data is in the lower right of the table and all annotations appear above or to the left of the experimental data.

In addition to the experimental data to be graphically represented on the display, all other data associated with the experimental data is also loaded and correlated into the system. For example, p-values, error analysis statistics, and other associated values may be loaded. Any ratio-based data or other data represented by numerically orderable measurements may be graphically represented and manipulated according to techniques described in the present specification.

Non-experimental data such as that displayed in rows R1- R4 can be loaded in a normalized scheme, in step S3 in an "n x y" matrix, where "n" is a positive integer representing the number of columns in the matrix, which will be displayed as an extension of the columns displaying the experimental values of the n x m matrix, and "y' is a positive integer representing the number of rows in the matrix. The "n value" (i.e., n = 1, 2, 3...n) of each column of the n x y matrix is linked to the corresponding "n value" in the n x m matrix in step S5, so that when a column of the experimental data is reordered by a sort, the column in the n x y matrix which corresponds to the column of experimental data that is reordered is reordered along with it to maintain the proper identification of each column of experimental data by the correct non-experimental data. This linking may be accomplished via BNS-like mechanisms that can match up identifier schemes (even when they are different, as long as a mapping between them exists). In some simple cases the identifiers may be consistent between the two data sets and it is only required that the identifier column is known. This may be by convention (e.g., the first column of every table must be a gene identifier derived from Unigene). Another way of accomplishing the linking it to require the user to identify the column to be used for linking, at the time that the data is imported for use by the present system in creating a display and manipulating the data displayed therein. Still another technique for linking is to program the software to analyze the data as it is imported and determine if a column contains recognizable identifiers. For example, the system may scan all the data during import and determine that all entries in a particular column have a recognizable identifier (e.g., all entries in column two start with "Hs.") and so are probably Unigene identifiers and can be used to accomplish the linking. Another example is that all entries may start with "NM_" and so are refseq mRNA identifiers, which can be used as a basis for the linking. Although the last technique described is highly domain specific, it provides useful functionality for users in that domain.

It should be further noted that steps S3 and S5 are optional, i.e., the present invention can display experimental data and reorder the data as described herein without the necessity of including non-experimental data in rows corresponding to the experimental data. The rows of non-experimental data however, when available, add further information to be viewed by the user in a single display.

Similarly, in step S7, non-experimental data such as that displayed in columns 42, 44, 46, 48, 50 and 52 in Figs. 4-6, for example, can be loaded in a normalized scheme, in a "z x m" matrix, where "z" is a positive integer representing the number of columns in the matrix, and "m" is the number of rows of the matrix, which will be displayed as an extension of the rows displaying the experimental values of the n x m matrix. The "m value" (i.e., m = 1, 2, 3 ... m) of each row of the z x m matrix is linked to the corresponding "m value" in the n x m matrix in step S9, by techniques similar to those described above with regard to column linking, but with common row identifiers, so that when a row of the experimental data is reordered by a sort, the row in the z x m matrix which corresponds to the row of experimental data that is reordered is reordered along with it to maintain the proper identification of each row of experimental data by the correct non-experimental data. It should be further noted that steps S7 and S9 are optional, i.e., the present invention can display experimental data and reorder the data as described herein without the necessity of including non-experimental data in columns corresponding to the experimental data. The columns of non-experimental data however, when available, add further information to be viewed by the user in a single display.

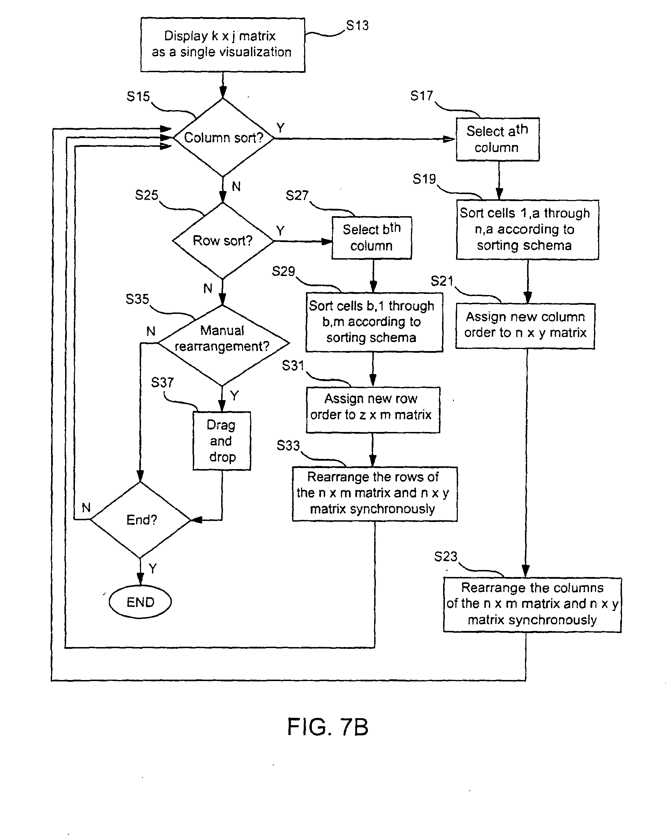

After constructing the underlying matrix as described above, which serves as the basis for displaying the visualization 100, the data from the matrix is displayed in a single visualization made up of a k x j matrix (step S13, Fig. 7B). The k x j matrix will generally be limited by the capacity of the monitor or display upon which the visualization is outputted, and may be predetermined by the display software. It is generally preferable to display as much data as can be reasonably viewed by the user without over-taxing the eyesight, and it is generally preferable, although not absolutely necessary, to display all of the non-experimental data and all of the columns of the experimental data, so that, for example, in Figs. 4-6, at least a portion of the data from each microarray is visible. According to this preference, "k" would be a positive integer equal to the sum of "n" and "z", i.e., k = n + z. Note that some or all of the non-experimental data may need to be abbreviated or cut off, but a tooltips feature may be provided so that when a user hovers the mouse sprite over a compressed, abbreviated or cut-off representation of non-experimental data in a cell, a pop-up display of the full expression of the non-experimental data is displayed. Also, if "n + z" is a value greater than a preset maximum value for "k", then some of the columns of the experimental data may not be displayed, although these values will still be considered in performing manipulations and they may be displayed upon reordering of the columns of experimental data. As to the number of rows displayed in the visualization, the display will be generally inadequate to display all of the rows in examples where the experimental data represented is microarray data or protein abundance data for example. In these instances "j" is an integer equal to the number of rows that can be reasonably visualized on the display and can be preset in the software, but will be less than the sum of "m + y". Generally, the system is arranged so that all of the rows of non-experimental data is displayed, while only a first portion of the "m" rows of experimental data is displayed. The experimental data and non-experimental data in rows higher than "j" are accessible by the manipulations of the data, but will only be displayed upon reordering, when one or more rows of the experimental data has been determined by a sort to be of particular interest. The situation where not all columns of experimental data can be displayed does not occur as frequently as the situation when not all the rows may be displayed. For example, when considering microarray data, each column pertains to a microarray and the number of microarrays to be considered can be easily controlled by the user.

Upon viewing the display 100, if the user decides to perform a column sort at step 515, then the user outlines a row of the experimental data display 110 in step S17 (i.e., the $a^{th}$ row of the total "m" number of rows, where "a" can be any integer from "1" to "j" of the experimental data) which contains data of interest upon which the user desires to perform the column sort. The outlining may be accomplished by aligning the cross hair 114 as described above, or by other visual indicating means. Upon selecting the $a^{th}$ row, as described, each experimental data value (i.e., cells one through n of the $a^{th}$ row, noted as cells 1,a through n,a in step S19)) are compared to perform a new sorting order, whether the cells are to be arranged in descending order of value or ascending order of value. This sorting schema is an iterative process in which the first cell is compared with the second to determine the sorting arrangement and then either the first or second cell, whichever is determined to be of lower value according to the sorting schema is compared with the value of the third cell, and so forth, and can readily be accomplished by one of ordinary skill in the art. It is important to note, however, that cells one through z of the $a^{th}$ row of the z x m matrix are not considered or compared during the sorting procedure, as they contain non-experimental data that would be meaningless or erroneous to compare with the experimental data values during the sort.

After completing the sorting procedure, the cells in the $a^{th}$ row are assigned their new column order designation, and all cells in each column of the n x m matrix are assigned the same new column number as the cell in the $a^{th}$ row that they share a column with. Also, in step S21, the columns of non-experimental data in the n x y matrix are reassigned new column numbers that correspond to the new column numbers of the experimental data columns that they are linked with. In step S23, the columns of the n x m matrix and the n x y matrix are rearranged or reordered synchronously to be visually displayed in the display 100 according to the new ordering scheme.

If the user decides to perform a row sort at step S25, then the user outlines a column of the experimental data display 110 in step S27 (i.e., the $b^{th}$ column of the total "k" number of columns displayed, where "b" can be any integer from "1" to "k") which contains data of interest upon which the user desires to perform the column sort. The outlining may be accomplished by aligning the cross hair 112 as described above, or by other visual indicating means. Upon selecting the $b^{th}$ column, as described, each experimental data value (i.e., cells one through m of the $b^{th}$ column, noted as cells b, through b,m in step S29) are compared to perform a new sorting order, whether the cells are to be arranged in descending order of value or ascending order of value. This sorting schema is an iterative process like the one described above with respect to the column sort. It is important to note, however, that cells one through y of the $b^{th}$ column of the n x y matrix are not considered or compared during the sorting procedure, as they contain non-experimental data that would be meaningless or erroneous to compare with the experimental data values during the sort.

After completing the sorting procedure, the cells in the $b^{th}$ column are assigned their new row order designation, and all cells in each row of the n x m matrix are assigned the same new row number as the cell in the $b^{th}$ column that they share a row with. Also, in step S31, the rows of non-experimental data in the z x m matrix are reassigned new row numbers that correspond to the new row numbers of the experimental data rows that they are linked with. In step S33, the rows of the n x m matrix and the z x m matrix are rearranged or reordered synchronously to be visually displayed in the display 100 according to the new ordering scheme. The user can choose to manually reposition (step S35) one or more columns or rows by dragging-and-dropping row(s) and/or column(s) at step S37, in the manner described above.

Similarity Sorting

The column, row and manual sorting procedures described above can be useful in identifying correlations, trends and other relationships among the data in some instances. However, when dealing with large volumes of experimental data, such as microarray data sets or protein or other molecular data sets, the data sets are often sufficiently "noisy" that it is often difficult to find meaningful correlations by simply sorting a single column (e.g., a single array) or a single row (e.g., a single gene). When experimental data such as these are measured by very low level signals, there may be a lot variation in the measured values from experiment to experiment and they are inherently "noisy'. Microarrays are generally noisy due to a number of experimental variances. Microarrays are generally qualitatively reproducible, but the individual measurements will still show quite a bit of variance. Thus, if a sort is performed on the basis of a single or individual array, slightly different ordering results are observed, as compared to the same sort performed on an array which is already known to be similar. These differences may even occur when a sorting procedure is performed on two different arrays representing the same experiment (i.e., a replicated experiment) due to differences in noise levels between the two arrays. To address these problems, the present invention further provides the capability of performing similarity sorting, which includes the ability to sort the data set by row or column similarity.

Similarity sorting of a row differs from the standard row sorts described above, in that a similarity calculation is performed between a selected row of experimental data and each non-selected row of experimental data to compare each entire non-selected row to the entire selected row to determine how close or similar it is to the selected row, and then the rows are ordered in terms of their similarity ranking with respect to the selected row, which assumes the position of row 1. As to similarity column sorting, an entire selected column of experimental data is compared with each entire non-selected column of experimental data to determine similarity rankings and the selected row assumes column 1 with the remaining columns following in position according to their similarity ranking. The rows and columns of non-experimental data are treated in the same manner that they are treated for standard row and column sorts, so as to maintain association with the appropriate rows and columns of experimental data.

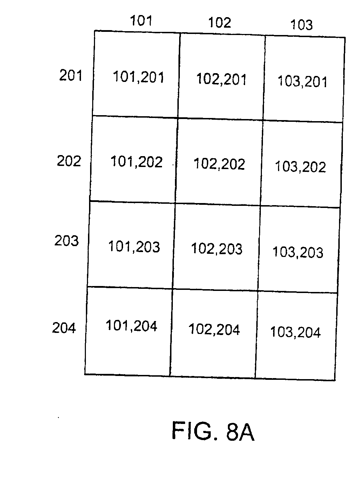

Fig 8A shows a simple 3 x 4 matrix which will be used to refer to a very simple demonstration of similarity sorting according to the present invention. The 3 x 4 matrix represents and experimental data set, i.e., an "m x n" matrix as described above with regard to Figs. 7A-7B. Of course, the actual experimental data sets which will generally be treated by the present system and methods will be much larger, such as the 31 x 8,066 matrix referred to in the examples above, but a 3 x 4 matrix has been shown to greatly simplify an explanation of the procedures, while at the same time, explaining the concepts and techniques required, which can then be readily applied to larger data sets.

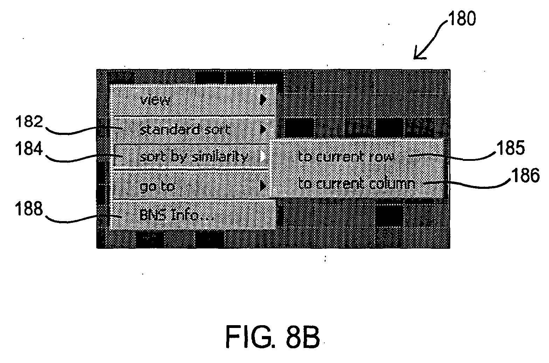

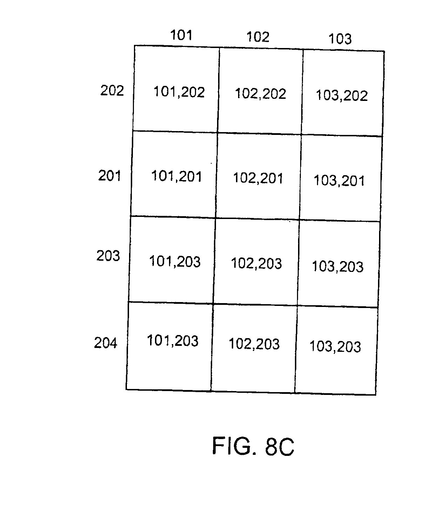

A similarity column sort or similarity row sort may be performed on any of the columns (101, 102, 103) or rows (201, 202, 203, 204) that the user so selects. Thus, for example, assume a user wishes to perform a similarity sort on row 202. By selecting row 202 in Fig. 8A, such as by using the cross hair 114 or other indication means, such as by right clicking on a column or row header or cell representing an experimental data value, the system invokes a popup menu 180, as shown in Fig. 8B. Popup menu 180 gives the user options, among others, of performing a standard sort or a similarity sort. In the view shown in Fig. 8B, a similarity sort has been selected, and the system at this time provides further options as to whether the similarity sort is to be performed according to the current row selected 185 or current column selected 186. Although not shown, selection of a standard sort would provide the same options (i.e., as to row or column based sorting), and sub-sorting as well as next neighbor sorting options may also be provided in the popup menu 180 or a similar popup feature. After selecting a similarity row sort in this example, the system rearranges the matrix of experimental data such that row 202 becomes the first row positioned in the matrix as shown in Fig. 8C. Any non-experimental data (e.g., data in the z x m matrix characterizing rows 201 and 202 (which happen to be the only two rows that were repositioned at this stage) is repositioned so as to maintain the positions relative to the experimental data prior to the row reordering. The experimental values expressed in the cells of the rows are then compared by a similarity test, to determine the relative similarity of each of rows 201, 203 and 204 to row 202. One method of determining relative similarity is to calculate the squared Euclidean distance of each row 201, 202, 203 from row 202 and then sort the rows 201, 202, 203 according to the squared Euclidean distance, with the row having the smallest squared Euclidean distance being positioned adjacent row 202 and the row having the next smallest squared Euclidean distance from row 202 being positioned adjacent that column, with the largest distance in this example being ordered as the last row.

In the example chosen in Fig. 8C, the squared Euclidean distance between rows 202 and 201 would be calculated as follows:

$$D(202,201) = ((101,202) - ( 101,201 ))^2 + ((102,202) - (102,201))^2 +$$

$$((103,202) - (103,201))^2$$

Where:

D is the squared Euclidean distance value;

D(202,201) represents the squared Euclidean distance value between rows 202 and 201;

(101,202) represents an experimental data value in cell 101,202 of row 202 that is being used for purposes of determining similarity;

(101,201) represents an experimental data value in cell 101,201 that is being used for purposes of determining similarity; and so forth.

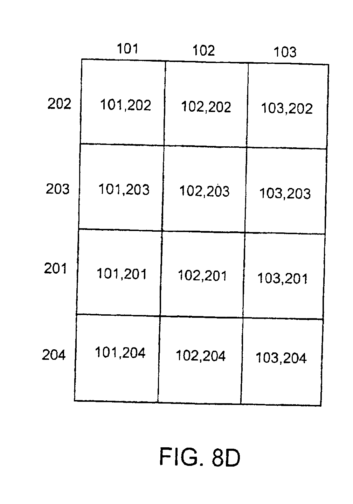

After determining D(202,201), D(202,203) and D(202,204) are calculated using the same approach. The values of D(202,201), D(202,203) and D(202,204) are then compared to rank order them with respect to row 201. The lowest value determines the next row to be positioned immediately beneath row 201, with the second lowest value being placed beneath that, and so forth. Thus, in the above example, assuming that the calculated value for D(202,203) is less than the calculated value for D(202,201) which is less than the calculated value for D(202,204), i.e., D(202,203) < D(202,201 ) < D(202,204), then the reordered matrix according to the similarity row sort described would appear with row 202 in the top row, followed by rows 203, 201 and 204, in that order, as shown in Fig. 8D. Similar to the standard row sorting, any cells containing non-experimental data adjacent the rows 201-204 are not considered for the Euclidean distance calculation (or any other similarity algorithm that may be employed). However, the adjacent, non-experimental data that is linked with these rows is reordered respectively with the reordering of the experimental data in those rows to maintain the normalized schema.

Alternatively to Euclidean distance, other measures of similarity may be performed in conducting similarity sorting as described above. For example, an alternative distance based on the Pearson correlation coefficient may be computed as follows:

$$r = \frac{\sum X_i Y_i - \dfrac{\sum X_i \sum Y_i}{N}}{\sqrt{(\sum X_i^2 - \dfrac{(\sum X_i)^2}{N})(\sum Y_i^2 - \dfrac{(\sum Y_i)^2}{N})}}$$

where X = a first column or row being considered for similarity measurement,

   Y = a second column or row being considered for similarity measurement,

   N = the total number of X or Y values in a column or row X or Y, and

the distance is measured as I -r.

The Euclidean measurement technique described may be desirable for finding rows (or columns) which are closely similar in overall amplitude, while the Pearson correlation coefficient may be more desirable for sorting a separating correlated and anti-correlated rows (or columns), though similarity in this approach is weighted more toward the overall pattern or shape of an expression profile, rather than its amplitude. In any case, the user may select among similarity measurements and may choose to approach the data with more than one type of similarity measurement, to compare and contrast the results achieved.

A similarity column sort would be conducted in a very similar manner to that described above with regard to a similarity row sort. The column selected by the user would be repositioned in the first or leftmost column and then similarity calculations would be conducted between experimental data in the selected column and each remaining column of experimental data to determine a reordering of the columns by their similarity to the selected column. Similar to the standard column sorting, any cells containing non-experimental data adjacent to the columns 101-103 would not be considered for the Euclidean distance calculation (or any other similarity algorithm that may be employed). However, the adjacent, non-experimental data that is linked with these columns would be reordered respectively with the reordering of the experimental data in those columns to maintain the normalized schema.

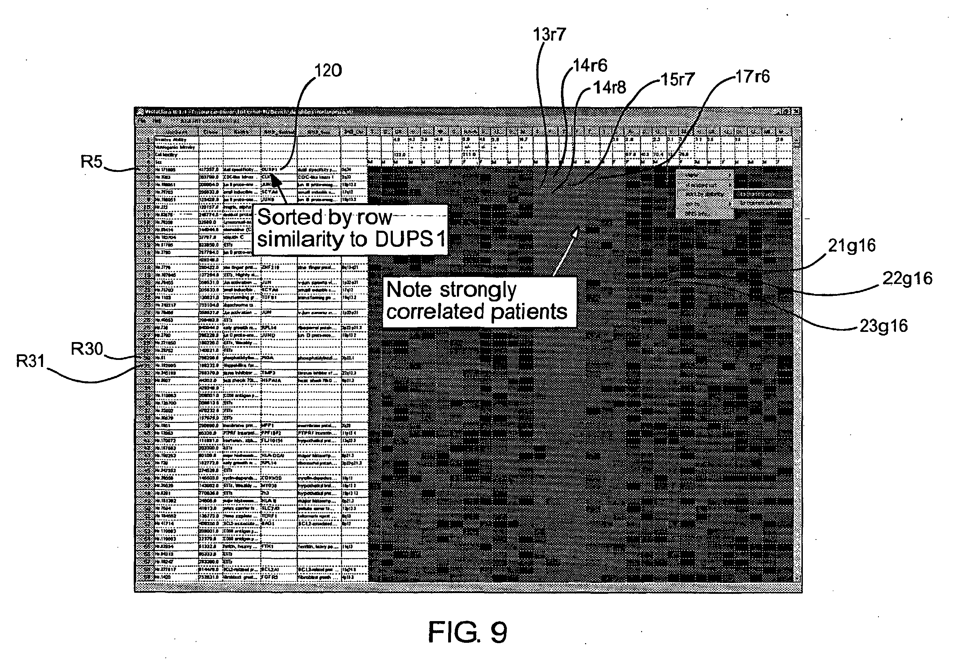

It should be noted that since the similarity reordering is done based upon similarity to the selected row or column, not all adjacent rows are necessarily most similar to one another. This is especially true as the number or rows or columns increases. Thus, for example, in Fig. 9, the results of a similarity row sort based upon the gene "DUSP1" (selected row appears in row R5) is shown. In this case, for example, rows R30 and R31 aren't necessarily very similar to each other, as they are ranked based on their similarity to row R5. Rather, what the order indicates is that the gene expression values in row R30 are more similar to those in row R6 than the similarity between row R31 and row R6, i. e., D(R6,R30) < D(R6,R31). However, the overall result of such a sort reorders the genes based on their aggregate similar behavior across many microarrays in the case of microarray experimental data.

When calculating the squared Euclidean distances, there are several considerations that apply to the present procedures that do not necessarily apply generally to the calculation of a Euclidean distance between two points of data in any Euclidean space. With regard to microarray experimental data, all distances are computed in log space to avoid biasing toward up-regulated genes. With two dye microarrays, data are generally stated as ratios of some sample treatment relative to some standard. If the data are expressed as a simple ratio, then values are always positive with up-regulated ratios being greater than one and down-regulated ratios having a value less than one but greater than zero. A simple example will confirm that the use of such ratios would tend to overweight up-regulated genes when determining D. For example, assume in Fig. 8C that the expression values of each of cells (101,202) and (102,202) is one, i.e., normal or neutral, that the expression value of cell (101,201) is 2x down-regulated, i.e., has an expression ratio value of .5, and that the expression value of cell (102, 201) is 2x up-regulated, i.e., has an expression ratio value of 2. If we consider the squared distance contribution between cells (101,202) and (101,201) as well as the squared distance contribution between cells (102,202) and (102,201) as would be done in the course of determining an overall squared distance value between rows 202 and 201, we obtain the following:

$$((101,202) - (101,201))^2 = (1 - .5)^2 = .25$$

$$((102,202) - (102, 201))^2 = (1 - 2)^2 = 1$$

Thus, it can be seen that the overall contribution to the sum of the squared distances which determines the similarity between the entire rows, is much more heavily weighted by the up-regulated gene expression ratio, even though the down-regulated ratio is separated from a "normal reading" by the same factor (2x) as the up-regulated expression ratio. To eliminate this biasing factor, log ratio expression data is used in the similarity calculations, or if expression ratio data is displayed, then the expression ratio data is first converted to log expression ratio data. By using log expression ratio data, both up and down regulated genes are symmetric with respect to absolute magnitude and no bias towards up-regulated genes occurs in the similarity calculations.

Another consideration is that a true Euclidean distance is measured by the square root of the sum of the accumulated squares of the measurement differences taken. However, since the goal of the procedures according to the present invention is only to determine a relative sorting value of rows or columns based upon relative distance to a selected row or column, and not to determine actual distances from the selected row or column, the sum of the squared differences between corresponding cells is sufficient, and the square root of the sum need not be determined. Since the same relative results can be determined without calculating the square root values, the square root calculation may be dispensed with.

In calculating differences between corresponding cells, differences involving cells that have invalid or missing data are treated as if the difference is 0 so that it does not unduly contribute one way or the other to the overall value of D used in determining similarity. In effect, this treatment defaults to assuming similarity of the missing data, rather than imposing some exaggerated notion of arbitrary dissimilarity in these instances. Although this treatment may give somewhat less than precise results, the situations where data is invalid or missing in a cell must be addressed in some fashion, and as long as the amount of missing data is small in comparison to the rest of the distances calculated, the effect is negligible. Alternative ways of addressing these situations include allowing the user to select what action to take in such cases and either use the current approach or eliminate the data, or treat the distance as some fixed value other than zero.

In the unlikely event that the D values for two rows or columns turn out to be equal, the ordering of these two rows with respect to one another is arbitrary (having been determined to both have equal similarity to the selected row/ column) and therefore the system arbitrarily places the lower numbered row or column nearer to the selected row or column, with the second row or column having the equal similarity value following. It is further noted that the similarity sorting procedures described above are only one approach to reordering data based on similarity among entire rows or columns of data. Various other approaches to manipulating the experimental data based upon characteristics of entire rows or columns may be readily applied by the instant invention. As just one further example, a similarity sorting order can be computed to group "nearest neighbors" of rows or columns. According to this approach, the selected row or column is positioned first followed by the row or column with the shortest squared Euclidean distance or other lowest valued sorting criteria (i.e., nearest neighbor). The third row or column is selected based on its determination as the nearest neighbor to the second row or column and positioned adjacent thereto, and so forth. According to this procedure, all rows or columns are calculated for similarity or proximity to the selected (first positioned) row or column, just as in the above-described procedure, to determine positioning of the second row or column. However, this approach varies for placement of the third and subsequent rows/columns. For the second and subsequent row/column positions, the distance/proximity calculations are repeated or iterated wherein the row/column positioned just filled is treated as the selected row/column. For example, for placement of the third row/column, the second placed row or column is used to determined distances/proximities with respect to all remaining rows/columns except the first row/column which has already been placed. By this iterative treatment of the data, what results is an ordering wherein the second row/column is the nearest neighbor of the first row/column; the third row/column is the nearest neighbor of the second row/column; the fourth row/column is the nearest neighbor of the third row/column, and so forth, as contrasted with the previously described procedures where each row/column is ordered based upon its relative similarity to the first column/row. By this approach, each adjacent row/column is positioned so as to be relatively similar to its neighbors and

this provides an additional view by which the user might identify emerging trends among the experimental data.

It should be further noted that similarity sorting using the squared Euclidean distance between the selected column or row and the remaining columns or rows is only one algorithm that can be employed in determining similarity sorts (according to a selected column/row, by nearest neighbor, or otherwise) by the entire row or column. Many other algorithms, measures and schemes may be used to accomplish a reordering of the experimental data based upon entire rows/columns cumulatively. For example, weighting factor(s) based on experimental error statistics could be used so that very noisy measurements don't contribute to the overall measure as much as more reliable data. Similarity measures that utilize more than one data type for performing similarity computations may also be employed (e.g. combine microarray-generated ratio data with TAQMAN measurements, etc.). Other techniques readily suggest themselves, and standard data-mining techniques and algorithms can be applied to sort rows and columns by various criteria. However, the key property of such sorting should be that it's fast enough to be reasonably interactive to allow for user directed data browsing. If the computation is too time-consuming then it should be performed by more traditional non-interactive modes of data mining. A significant advantage of the current algorithms implemented is that they are very fast to compute and thus are virtually as interactive as a typical column sort.

Another variation for performing similarity sorting is to allow user selection of the distance measure. For instance, the user might chose as an option to calculate squared Euclidean distance with or without error weighting. Another option provides an embedded scripting environment that allows the user to design a custom measure scheme, which would then become one of the optional methods. Other similarity algorithms may alternatively be employed to determine a similarity ranking for display of the experimental data according to the present invention.

Further, although the examples above describe performing the similarity sorts based upon the displayed experimental data values (such as the gene expression values displayed by color-coding in the example of microarray data), similarity sorting can also be accomplished based upon other values associated with the experimental data values that are primarily displayed in the matrix. These types of sorts can be accomplished as a primary sort to display similarity of the experimental data based on the associated values, or can be accomplished secondarily to a similarity sort performed first by using the displayed experimental data values. For example, in the case of microarray data, a similarity sort may be performed based upon the displayed gene expression ratios, after which a further similarity sort (based on the same selected row or column) may then be performed based on error statistics, p-values, standard deviations, or other secondary data types associated with the expression ratios, wherein the values of the secondary data type selected are used to determine squared Euclidean distance values or other similarity sorting values.

Sub-Sorting

To further extend the flexibility and versatility of the present invention for providing various arrangements of experimental data likely to expose trends, correlations or other relationships among the experimental data when viewed by a user, the experimental data may be sub-sorted either after performing any of the sort procedures described above or even initially after displaying the experimental data as loaded. The sub-sorting procedures may be the same as described above with regard to any of the sorting procedures. Sub-sorting procedures differ from those described earlier in that the row or column selected by the user for sub-sorting is not re-positioned to the first row or column space of the matrix 110. Rather, the selected row or column maintains its current position upon selection, and only rows/columns subsequent to this position are considered for the sub-sort (i.e., rows below the selected row or columns to the right of the selected column). The previous rows or columns are left in the same positions as prior to the sub-sort procedure and are therefore unaltered by the sub-sort.

The user interaction for performing a sub-sorting procedure is effectively the same as described above with regard to various methods of similarity sorting, except that upon selecting a row or column, the user chooses the sub-sort function, and specifies a row-based or column based sub-sort, whereby the selected row/column maintains its present location and the subsequent rows/columns are reordered based upon similarity calculations carried out. By iteratively using this sub-sorting method in conjunction with standard sorting procedures, the user can create ad-hoc groupings of similar matrix elements. This has the effect of something similar to user-directed clustering, but is much less mathematically rigorous and therefore much faster for real time interactive use. The groupings have meaning only to the user constructing them and care must be taken not to over-interpret what they signify. Still, they may provide insight into the relationships within the underlying data, or at a minimum provide some method for mathematically grouping related items.

Graphical Representations of Experimental Data

Alternative to the graphical representations of experimental data referred to, shown and described above, the present invention provides alternative methods and visualizations for the graphical representation of experimental data. Although the following examples refer to alternatives for representing microarray data, the alternative techniques are not to be limited only to gene expression values and other data represented by microarrays, but may be extended to data sets of other experimental data as well, including protein abundance data, or any other numerically sortable data that can be represented as a heat map.

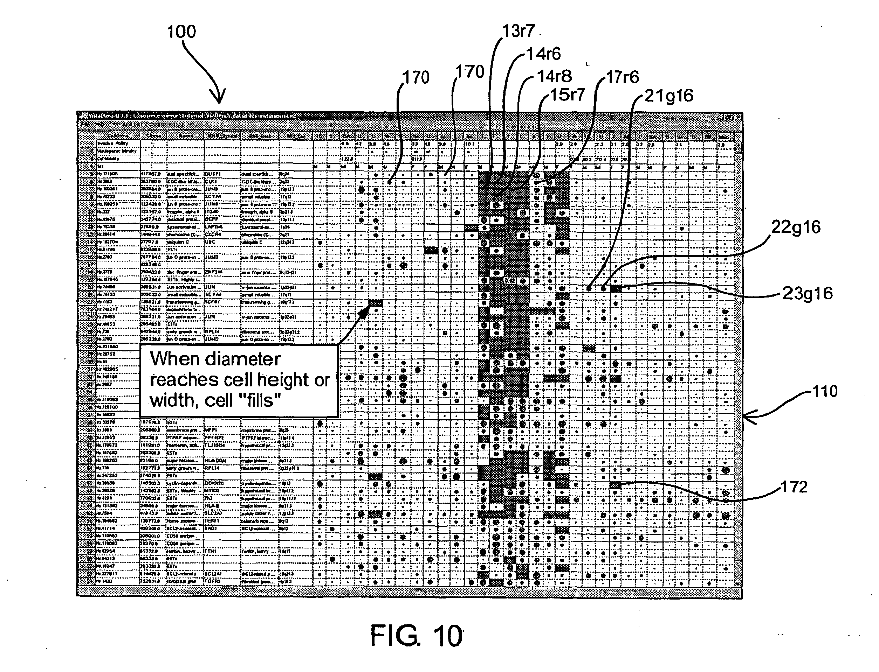

Fig. 10 shows a visualization 100 that employs an alternative representation of the traditional heat map view in the experimental data portion 110 of the matrix 100. This visualization of experimental data is based on a technique to more graphically represent a relative quantification of the underlying expression values represented by the graphical display. Based on observations that the standard color indicators and their various shades are not as effective for a user to visually identify or interpret relative quantity values of the underlying expression data as would be some graphical indication of relative size, the present invention in Fig. 10 utilizes a relative sizing scheme in addition to the standard color/shading gradients scheme for graphically displaying the expression data in matrix 110. In the example shown, circles 170 of varying size are displayed to graphically convey the relative values of the quantitative data (in this case gene expression ratios). Thus, the size of the circles 170 varies according to the magnitude of the value being represented. Color-coding is also employed both to differentiate between down-regulation( e.g., green color-coding) and up-regulation (e.g., red color-coding), as well as to show relative values by use of gradients in the two basic colors, with black being displayed from neutral, as is common in standard heat maps. These color concepts, as well as graphical size indicators, can be applied to differentiate between negative and positive data values with regard to data sets other than microarray gene expression data as well. In any case, the use of size indicators, such as circles 170 provides the user with a more readily perceptible differentiator between magnitudes of values being represented, particularly those that are somewhat close to one another that may be more difficult to detect by perceiving slight shade differences of red or green, for example. Used together with color-coding, the graphical size indicators assist in identifying trends and similarities among the data, wherein the color-coding readily separates the data among negative and positive values, or up-regulation and down-regulation. The color gradients for differentiating closer values adds to the overall perceptibility of trends and similarities.

The diameter of each circle 170 shown in Fig. 10 is proportional to the absolute value of the underlying value. In determining the circle diameters, the value computed for the color-coding of each cell is also used to determine the diameter of the circle, so the same scaling is used. When computing the diameters of the circles for placement within the cells of matrix 110, if the diameter of any circle 170 is computed to be large enough to touch or intersect with the perimeter (either a horizontal line or a vertical line forming the perimeter of the cell) the graphical representation is automatically expanded so as to fill the entire cell, as shown at 172, for example. By such representation, the user can then quickly spot values that lie above some arbitrary threshold. The present inventors refer to the graphical representation employing various sized circles 170 and filled cells (rectangles or squares) 172 as in Fig. 10, as "inkblots", given their overall appearance and resemblance to such. This type of representation enables a kind of sub-visualization in which the user can focus only on the filled-in rectangles and look for correlations and trends just within the values that fill in as rectangles.

Further, by use of the color-coding, this visualization provides a kind of heat map representation within the inkblots. This enables potentially powerful but still intuitive ways to view and examine the data.

By providing the graphical size indicators (e.g., circles 170 and filled in rectangles 172), the inkblots are much more effective at conveying a visual indication of the underlying data than colored heat maps. By comparing Figs. 9 and 10, which display the same experimental data, but only by different graphical visualization schemes, it can be observed that some cells that appeared to be significantly colored, and thus similar to other highly regulated cells, actually have relatively small ratio values and are not as significantly regulated as the ordinary heat map might indicate. For example, in Fig. 9, the appearance of the red color shading of cell 17r6 appears to be fairly similar to the surrounding highly up-regulated cells 13r7, 14r6, 14r8 and 15r7. However, when these same values are represented by an inkblot visualization as in Fig. 10, it becomes readily apparent that cell 17r6 actually has a relatively small ratio value, as indicated by a mid-sized circle, while cells 13r7, 14r6, 14r8 and 15r7 are all completely maximized, filled-in rectangles. Similarly, in Fig. 9, the appearance of the green color shading of cells 21g16, 22g16 and 23g16 all appear to be significantly down-regulated by their green color-coding. However, when these same values are represented both as to size and color-coding by an inkblot visualization as in Fig. 10, it becomes readily apparent that cell 23g16 is actually much more significantly down-regulated than either cell 21g16 or 22g16. Inkblot visualizations, like heat map visualizations, also have the advantageous quality of being row and column neutral, meaning that they are just as useful in spotting trends in the vertical dimension as they are in the horizontal dimension, or even both at the same time, since there is no biasing in either the column or row directions/dimensions.

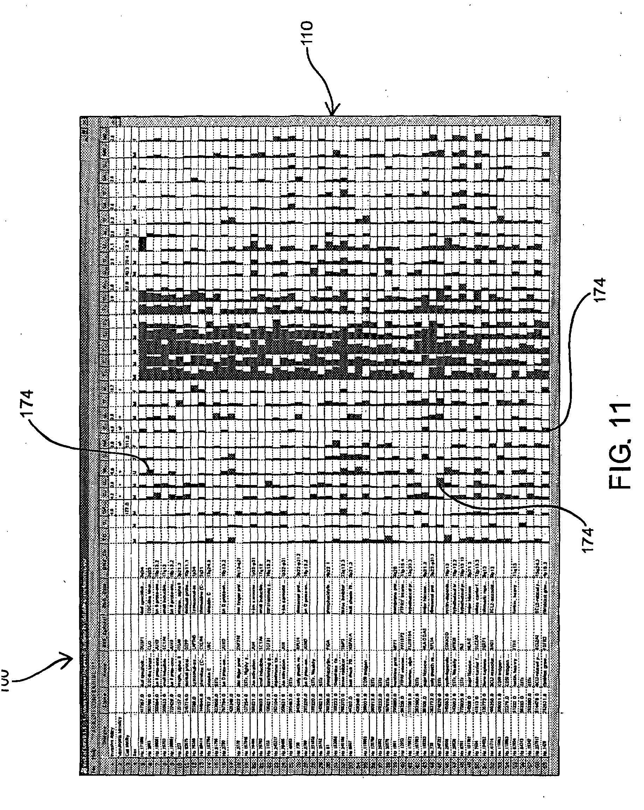

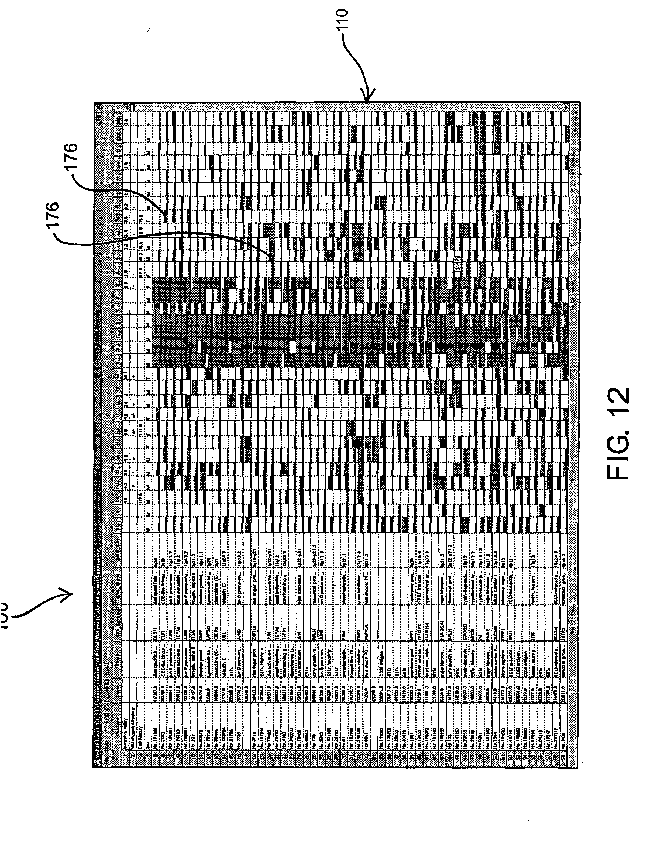

However, there may be situations when the data presents itself in such a manner that it would be advantageous to use a visualization that is biased toward either finding correlations among rows or among columns. The present invention includes further alternative visualization formats which are biased toward either spotting trends, correlations, etc in the horizontal direction, along which the rows of the matrix 110 extend, or toward spotting trends in the vertical direction, along which the columns of the matrix 110 extend. Advantageously, the present invention allows a user to switch the visualization format at will. For example, the experimental data may be originally visualized in the "heat map" style format shown in Figs. 4-6 and 9 (although this is not required, as any format may be used initially). The user may then wish to switch to an inkblot visualization (as in Fig. 10) as an aid to further sort what may be closely related data as indicated by the colors of nearby cells that may or may not have already been sorted by one or more of the sorting techniques described above. By viewing either of these visualizations with or without sorting while in either or both visualizations, the user may have some intuition of a trend or correlation developing either along columns or rows. In this instance, the user may further switch to a visualization as shown in either Fig. 11 or Fig 12, which are described hereafter. It should be emphasized again, that the visualizations of Figs. 11 and 12 need not be merely a secondary form of visualization, as the user may choose to display the data initially using either one of these formats, or an inkblot visualization. The present system provides complete flexibility to the user to determine the format of the visualization of the experimental data displayed, and the formats can be switched at will, in any order.

Fig. 11 shows a visualization 100 that represents the same experimental data in matrix 110 that is shown in Figs. 9-10, but employs an alternative representation of the graphical display of matrix 110. Like the inkblots visualization, this visualization of experimental data is based on a technique to more graphically represent a relative quantification of the underlying expression values represented by the graphical display. Unlike the inkblots visualization however, the graphical representation in this visualization is biased in the horizontal direction (along the rows of matrix 110). Fig. 11 utilizes a relative sizing scheme in addition to the standard color/shading gradients scheme for graphically displaying the expression data in matrix 110, but the relative sizing of the graphical representations in the cells varies only in the horizontal dimension, as the values are represented by horizontal bars 174 to give a horizontal bar graph or histogram appearance. Note that each bar 174 fills the entire vertical space of each cell, but varies as to the extent of the horizontal space that it fills. Like the circles 170 in the inkblot visualization, a horizontal bar will fill an entire cell when the absolute value of the data being expressed reaches a predetermined threshold value.

In the example shown, bars 174 of varying horizontal length are displayed to graphically convey the relative values of the quantitative data (in this case gene expression ratios). Thus, the horizontal length of the bars 174 varies according to the magnitude of the value being represented. Color-coding may also be employed both to differentiate between down-regulation( e.g., green color-coding) and up-regulation (e.g., red color-coding), as well as to show relative values by use of gradients in the two basic colors, with black being displayed for neutral, as is common in standard heat maps. The use of differential horizontally sized indicators 174 provides the user with a more readily perceptible differentiator between magnitudes of values being represented, particularly those that are somewhat close to one another in the horizontal direction and that may be more difficult to detect by perceiving slight shade differences of red or green, for example. Used together with color-coding, the graphical size indicators assist in identifying trends and similarities among the data, particularly within rows of data, since the size differentiators (varying lengths of bars 174) are oriented in the horizontal direction.

The length of each bar 174 shown in Fig. 11 is proportional to the absolute value of the underlying value, with the vertical dimension of the bar being equal to the height of the cell in which it is placed. In determining the bar lengths, the value computed for the color-coding of each cell is also used to determine the length of the bar 174 to be displayed in that cell, so the same scaling is used. When computing the lengths of the bars 174 for placement within the cells of matrix 110, if the length of any bar 174 is computed to be equal to or greater than the width of the cell in which it is to be displayed, then the graphical representation of the bar completely fills that cell to give the same appearance as a standard heat map representation in that cell. By biasing the visualization toward viewing in the horizontal or row direction, this potentially enhances the user's ability to identify trends correlations or other similarities across columns with regard to a particular row or rows of data. For example, when the data is microarray data, this visualization may provide enhanced insight into the comparative behaviors of a particular gene or group of genes across multiple arrays.

Fig. 12 shows a visualization 100 that represents the same experimental data in matrix 110 that is shown in Figs. 9-11, but employs another alternative representation of the graphical display of matrix 110. This visualization technique is very similar to the visualization in Fig. 11, except rather than differentiating the graphical bar indicators in the horizontal direction, the bars 176 in Fig. 12 are biased in the vertical direction (along the columns of matrix 110). Fig. 12 utilizes a relative sizing scheme in addition to the standard color/shading gradients scheme for graphically displaying the expression data in matrix 110, but the relative sizing of the graphical representations in the cells varies only in the vertical dimension, as the values are represented by vertical bars 176 to give a vertical bar graph or histogram appearance. Note that each bar 176 fills the entire horizontal space of each cell, but varies as to the extent of the vertical space that it fills. Like the visualization of Fig. 11, a vertical bar represented in Fig. 12 will also fill an entire cell when the absolute value of the data being expressed reaches a predetermined threshold value.

In the example shown, bars 176 of varying vertical length (i.e., height) are displayed to graphically convey the relative values of the quantitative data (in this case gene expression ratios). Thus, the height of the bars 176 varies according to the magnitudes of the values being represented. Color-coding may also be employed both to differentiate between down-regulation (e.g., green color-coding) and up-regulation (e.g., red color-coding), as well as to show relative values by use of gradients in the two basic colors, with black being displayed for neutral, as is common in standard heat maps. The use of differential vertically sized indicators 176 provides the user with a more readily perceptible differentiator between magnitudes of values being represented, particularly those that are somewhat close to one another in the vertical direction and that may be more difficult to detect by perceiving slight shade differences of red or green, for example. Used together with color-coding, the graphical size indicators assist in identifying trends and similarities among the data, particularly within columns of data, since the size differentiators (varying heights of bars 176) are oriented in the vertical direction.

The height of each bar 176 shown in Fig. 12 is proportional to the absolute value of the underlying value, with the horizontal dimension of the bar being equal to the width of the cell in which it is placed. In determining the bar heights, the value computed for the color-coding of each cell is also used to determine the height of the bar 176 to be displayed in that cell, so the same scaling is used. When computing the heights of the bars 176 for placement within the cells of matrix 110, if the height of any bar 176 is computed to be equal to or greater than the height of the cell in which it is to be displayed, then the graphical representation of the bar completely fills that cell to give the same appearance as a standard heat map representation in that cell. By biasing the visualization toward viewing in the vertical or column direction, this potentially enhances the users ability to identify trends correlations or other similarities across rows with regard to a particular column or columns of data. For example, when the data is microarray data, this visualization may provide enhanced insight into the comparative behaviors of a multiple number of genes in a single array or a group of related arrays.

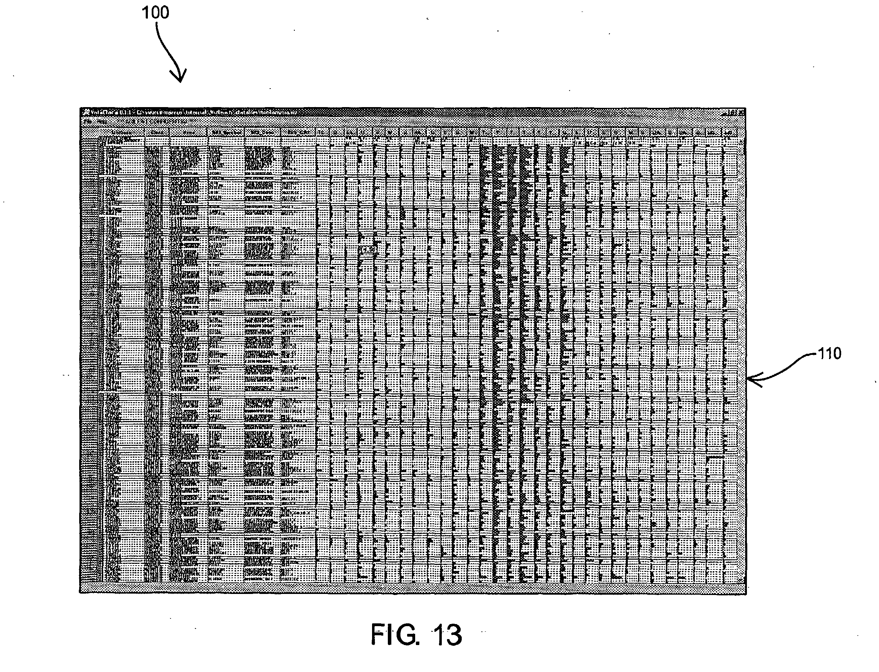

Fig. 13 shows a highly compressed horizontal bar graph, i.e., a visualization such as described above with regard to Fig. 11, except that the visualization has been compressed so as to maximize the number of rows of experimental data that can be visualized in the matrix 110 on a single screen. Note that the visualization has not been compressed to the extent that the data begins to overlap itself so that not all of the information can be displayed on the pixels of the visualization, such as in the case of the displays shown in Figs. 2-3, which use symbols to represent compressed groups of data and must be expanded to read individual items of data. In this way, a somewhat familiar format can still be presented to the user while increasing the amount of data presented on a single screen. That is, the data is still presented in a somewhat familiar style so that the user that is familiar with working with heat maps can use and interpret this type of display more intuitively.

With the present invention, a user can, at any time, select any of the above described visualization formats to use. Some data and/or some contexts might be more optimally viewed with one rendering style or another. Rather than impose a fixed rendering, the ability to choose a rendering is provided.

Additional Visualization Features

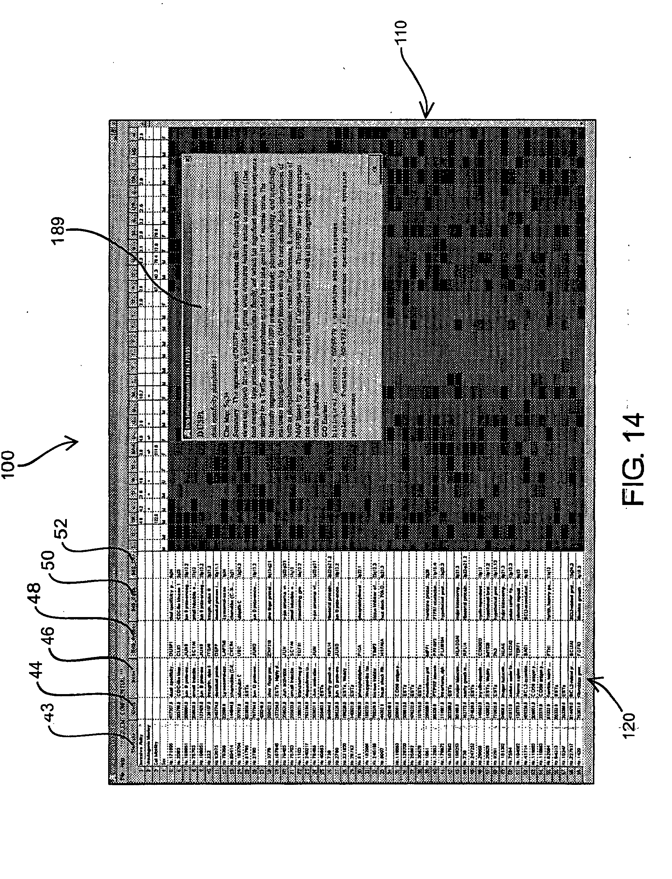

The present invention may be further linked with further sources of informational data to provide a more comprehensive characterization of the experimental data being examined. For example, each cell of the experimental data matrix 110 (or each cell of the entire matrix 100) may be linked to the biotechnology information naming system disclosed in co-pending and commonly owned Application Serial No. 10/154,529 titled "Biotechnology Information Naming System", filed on May 22, 2002 and incorporated by reference herein in its entirety. By right clicking on a cell of interest (experimental data cell), the popup menu 180 (Fig. 8B) appears, as described above. By selecting "BNS info" 188, the BNS system is accessed and information stored by the BNS system which describes the entity that the value of the cell of interest also describes is displayed in a popup dialog which can be viewed simultaneously with the selected cell. By selecting any cell in the matrix 110 (or any cell in the matrix 100 when all data, both experimental and non-experimental is linked), the system accesses a biotechnology information naming system server and attempts to look up any annotations contained in the server that are linked to the value of the cell in column 43 of matrix 100 (or other predesignated column known to have standard identifiers contained therein) that is also in the same row as the selected cell. In Fig. 14, the values contained in column 43 of the matrix 100 are Unigene ID values.

Upon retrieving pertinent annotations, these annotations are imported to the system and displayed in a pop-up window 189, as shown in Fig. 14. In the example shown, any cell contained within row R5 could have been clicked on to retrieve the annotations pertaining to DUSP1, which are displayed in the pop-up window 189. It should be noted that the annotations displayed in the pop-up window 189 are not part of any data set that the present system accesses to form the matrix display 100, but are retrieved from the biotechnology information naming system server when the user requests to see additional annotations about a cell that is selected.

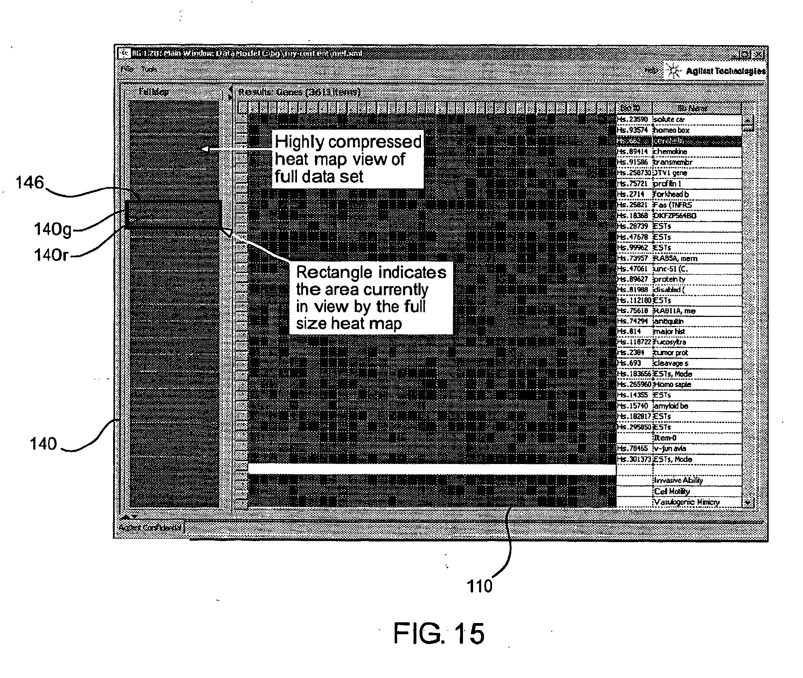

Fig. 15 shows a modified visualization according to the present invention which provides a generalized view of all of the experimental data in a compressed experimental data matrix 140, while at the same time providing an non-compressed view of a selected portion of the experimental data in matrix 110 in the same manner as described above. Although the non-compressed data in 110 is shown using a standard heat map style of graphical representation, this modified view is not limited to such, as inkblot, bar graph, histogram or other styles of graphical representation could be used instead. The various formats for graphical representation of the experimental data can also be switched in and out of at the will of the user, just as with the examples described above.

Although the view of Fig. 15 shows non-experimental data only in a matrix (i.e., a "z x m" matrix) adjacent the rows of the experimental data 110, it is noted that this visualization format is not limited to such placement of non-experimental data, as non-experimental data can be further included in an "n x y" matrix adjacent the columns of matrix 110, as in the previous examples. Also, although the matrix containing the non-experimental data in Fig. 15 is located to the right of matrix 110, this visualization format is not limited to such placement, as the non-experimental data could be located to the left of matrix 110 while placing the compressed matrix 140 to the right of matrix 110.

The graphical representation employed in the compressed view 140 is generally the same as that chosen for representing the non-compressed view 110. Although it would be possible to provide for independent selection of graphical representation formats for each of matrices 110 and 140, the present inventors have found that use of the same graphical representation style or format facilitates the ability of the user to identify correlations, trends or other similarities by additionally relating the non-compressed data and findings therein, with the compressed data and the location of the non-compressed data therein, as extension of a pattern, correlation or similarity may be extended within the entire data set 140 after identifying a relationship within the non-compressed data. Accordingly, the format displayed in 140 of Fig. 15 is the same standard heat map style representation, although on a greatly compressed scale. Due to the compression, individual cells are not represented in the matrix 140. However, the color-coding schema is maintained and therefore groupings of similar data may appear as predominantly red 140r or green 140g indications, for example.

The compressed view 140 is provided with a movable frame 146 that is scaled to outline a subset of the entire data set which corresponds to the capacity for display of non-compressed data by matrix 110. The frame is positionable by the user to capture any area within the compressed data 140 to be shown in a non-compressed view in matrix 110. The user may choose to perform any of the sorting techniques on the experimental data as described above with regard to the previous visualizations. As rows and columns are sorted in the full-size display 110, the same re-ordering of the experimental data is displayed in the full, compressed view 140. Thus, when rows that are not currently displayed in the non-compressed view 110 are brought into the non-compressed view 110 after performing a sort, the user may get a better overall understanding of the relationships between the data by having the ability to view the compressed colorized views of that data where it was located before being drawn into the non-compressed view. Thus, the compressed view 140 is re-ordered in real time upon reordering the non-compressed data 110 using a sorting technique. Thus, the effect of such interactive data mining can be seen for the same data set in the overall, compressed view at the same time that the user sees the effect on the data of interest in the non-compressed view 110. The blank row that is displayed in Fig. 15, is used as a separator to indicate that there are two different but related datasets being displayed in the matrix 110.

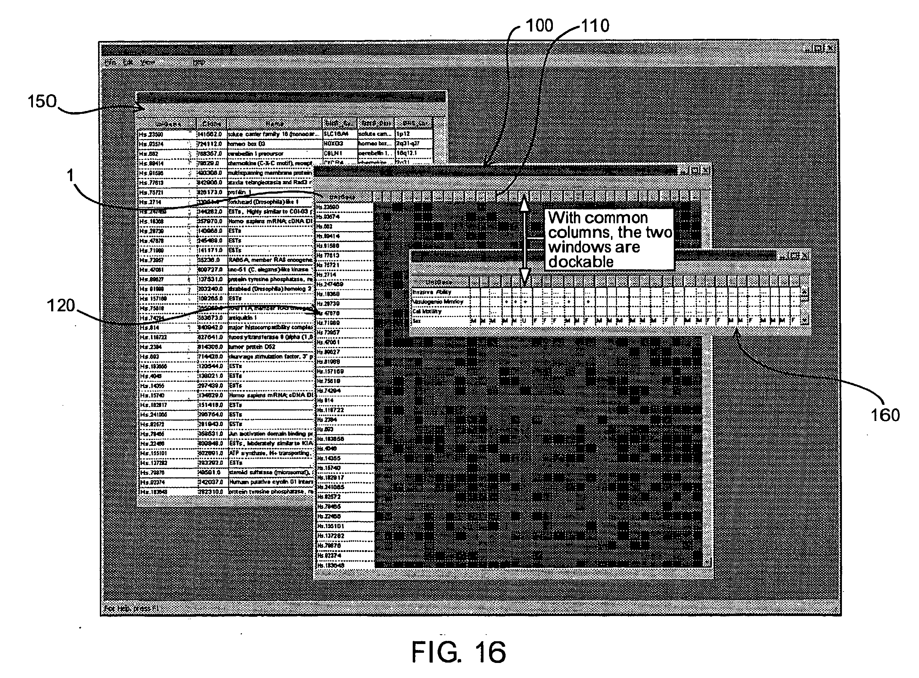

Turning to Fig. 16, a user interface mechanism is provided for combining related data of different types into a single unified visualization by the system according to the present invention. This feature provides for multiple independent data viewers, each dedicated primarily to a single data type. For example, the visualization in Fig. 16 employs three separate viewers, one for expression data 100 (i.e., experimental data), one for gene data 150 (i.e., non-experimental data) and one for clinical data 160 (i.e., non-experimental data). Note also that the viewer 100 also displays non-experimental data (i.e., Unigene data) in a z x m matrix 120 wherein z = 1 in this instance.

The interface mechanism allows docking of viewers which have the same or similar column or row headers. This mechanism operates on the same principle that allows Windows toolbars to be docked within a Microsoft application. Thus, when the user drags a viewer window near another compatible viewer window (i.e., another window having the same or similar column or row headers), the interface mechanism provides some visual indication (e.g., window frame edges that might join, may change color, frame edge style, flash, or otherwise indicate that the two matching edges are compatible for joining) that the two viewers are within a "dockable" region and can be joined. Alternatively the system may be designed to dock the two viewers without any visual pre-indication, as the viewers are approximated to relative positions that allow the docking to occur. The viewers can likewise be separated, or "snapped apart" by using the cursor to drag one window away from the other.

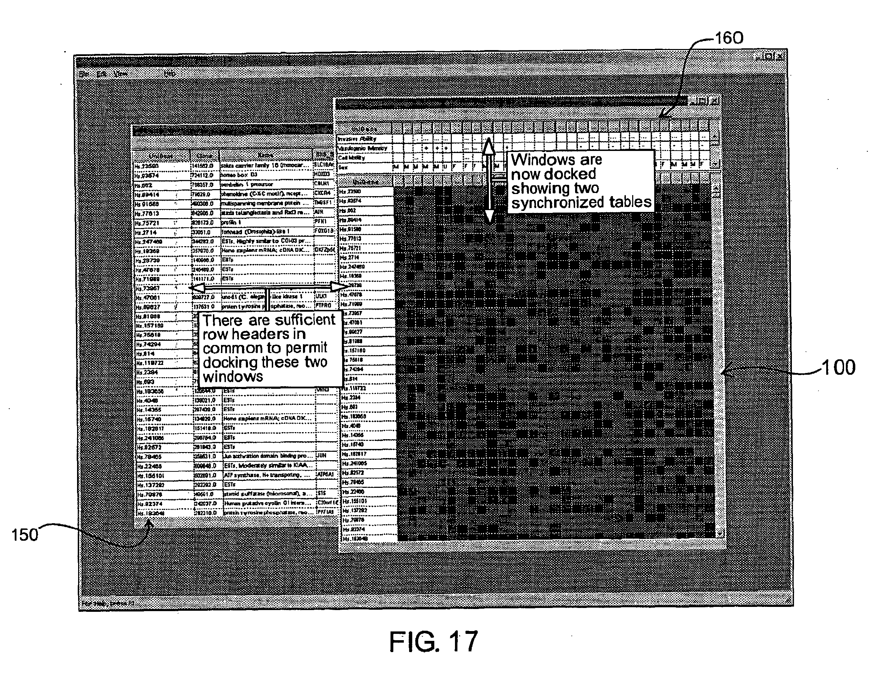

The clinical data viewer 160 and expression data viewer 100 share the same column headers, as shown in Fig. 17, and therefore may be dockable when stacked vertically. Thus, when the user drags the viewer 160 near the top of viewer 100 and releases or "drops" the viewer window 160, the two viewer windows 100 and 160 merge as shown in Fig. 17.

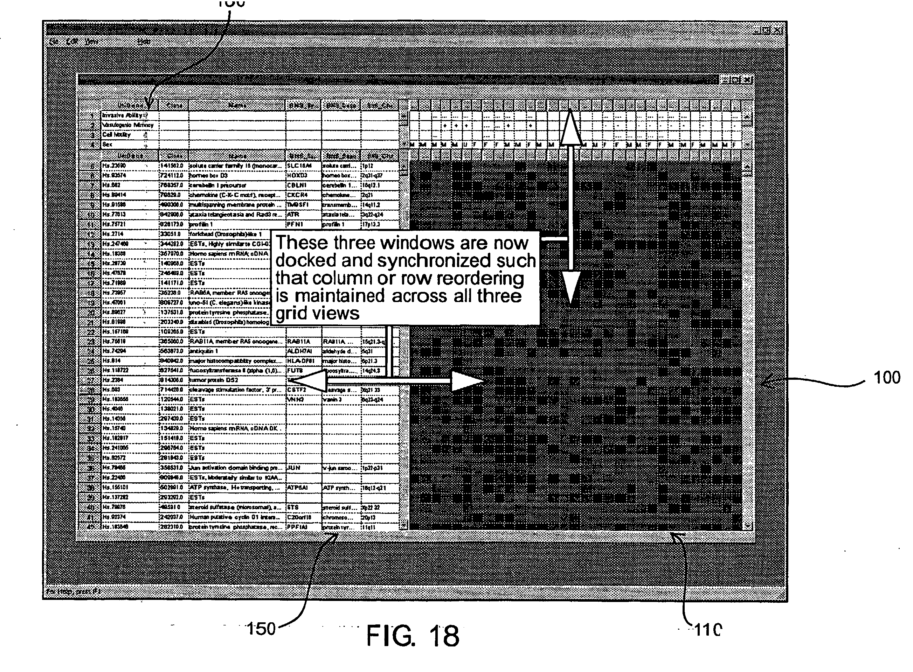

The gene data viewer 150 shares sufficient row headers that are the same as those in expression data viewer 100, as shown by the shared Unigene identifiers in Fig. 17. Thus, when the user drags the viewer 150 to the left side of viewer 100 and releases or "drops" the viewer window 150, the two viewer windows 100 and 150 merge as shown in Fig. 18. It is noted that two viewer windows do not have to have exactly the same row or column identifiers in order to be dockable, but only a significant number of shared column or row identifiers need be present in order to perform a docking operation that is useful. The definition of a significant number of shared identifiers may be arbitrarily set, but should be a high enough percentage so that the performance of the docking operation makes sense. When docking viewers 100 and 150 as described above, it would still be possible to perform this operation if, for example, viewer 150 lacked row identifiers (and data within those rows) for Unigene ID nos. Hs.2714, Hs.47678 and Hs.81988 (i.e., rows 13, 17, and 22 of the viewer 150). Upon docking the viewers, blank cells would simply appear in each of rows 13, 17 and 22, for each of columns 2-7, but the docked view would otherwise appear as it does in Fig. 18.

Further, the scroll bars and frames for viewers 110, 150 and 160 are maintained between the combined frames (Fig.18) so that the impression is still maintained that these are individual views that are simply docked and synchronized. By maintaining this separation, it is believed that this will more intuitively convey to the viewer which rows and columns will be effected by sorting operation (i.e., the experimental data cells in matrix 110), as distinguished from the cells that will merely be repositioned to track the movement of the experimental data cells as they are positioned based upon the sorting calculations or manual reordering that is performed with regard to these cells.

Using the mechanism described with regard to Figs. 16 - 18, the user can take advantage of some very simple drag-and-drop metaphors to intuitively build joined data sets, which provide synchronized scrolling, sorting and reordering. Using this mechanism does not require the user to resort to creating complex SQL queries involving joins, but rather they can simply associate windows which have obvious rows or columns in common.

While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, data type, manipulation, manipulation order, process, process step or steps, to the objective, spirit and scope of the present invention. All such modifications are intended to be within the scope of the claims appended hereto.

## Claims

1. A method for displaying and manipulating data to facilitate identification, trends, correlations or other useful relationships among the data, the method comprising the steps of:

   inputting (S1), into a memory of a processing system, data items associated with entities to be observed, wherein the entities are arranged in an n x m matrix, n equaling the number of columns in the matrix and m equaling the number of rows in the matrix;
   identifying a data item with respect to each entity that is to be represented in a display matrix, wherein each identified data item represents the same characteristic of each respective entity, although the value of each identified data item may vary;
   converting (S11) the identified data items to determine graphical representations of the identified data items to be displayed, wherein the graphical representations graphically represent variations in the values of the identified data items;
   displaying the first c x d graphical representations (110) of the identified data items in a c x d matrix, where c $\leq$ n and d $\leq$ m, with each graphical representation from the first c x d representations occupying a corresponding cell in the c x d matrix displayed;
   selecting a row (S17) or column (S27) in the displayed c x d matrix;
   sorting (S 19, S29) the order of arrangement of the entities in the n x m matrix based on a comparison of the values of the identified data items in the row or column of the n x m matrix corresponding to the row or column selected from the displayed c x d matrix;
   reordering (S21, S31) the order of arrangement of the entities in the n x m matrix based on the sort performed; and
   displaying the first c x d graphical representations of the identified data items from the reordered n x m matrix for observation by a user, for visual identification of changes in the relationships between the graphical representations.

2. The method of claim 1, wherein the sorting comprises rank ordering the identified items 1 through n, when a row is selected, which are assigned to the selected row, or rank ordering the identified items 1 through m, when a column is selected, which are assigned to the selected column, and wherein upon reordering, the column in or row which the first ranked identified data item is located is reordered to the first column or row position of the n x m matrix, the column or row in which the second ranked identified data item is located is reordered to the second column or row position of the n x m matrix and successive columns or rows are reordered according to the rank of the identified data item contained therein which also belongs to the selected row or column, wherein the first c columns or first d rows of the reordered m x n matrix are then displayed.

3. The method of claim 1, wherein the sorting comprises similarity sorting the rows or columns of the n x m matrix, wherein the selected row or column is reordered as row one or column one of the reordered n x m matrix and all other rows or columns are processed to determine a relative similarity value to the selected row or column, and wherein upon reordering, all other rows or columns are repositioned in descending order from row one or column one, based on ranking by the relative similarity values, and wherein the first d rows or c columns of the reordered m x n matrix are then displayed.

4. The method of claim 3, wherein a relative similarity value is determined by calculating a distance value between the selected row or column and a row or column to be assigned the similarity value, wherein each cell of the selected row or column is compared with a respective cell in the row or column to be assigned the similarity value.

5. The method of claim 1, wherein the sorting comprises nearest neighbor sorting the rows or columns of the n x m matrix, wherein the selected row or column is reordered as row one or column one of the reordered n x m matrix, all other rows or columns are processed to determine a relative similarity value to the selected row or column, the row or column having the nearest relative similarity value is reordered as row two or column two of the reordered n x m matrix, all remaining rows or columns which have not been reordered are processed to determine a relative similarity value to reordered row two or column two, the row or column having the nearest relative similarity to row two or column two is reordered as row three or column three, and this process is reiterated until all rows or columns have been reordered by nearest neighbor, wherein the first d rows or c columns of the reordered m x n matrix are then displayed.

6. The method of claim 1, further comprising the steps of :

   identifying a second data item, other than the data item which is graphically represented on the display, with respect to each entity in the n x m matrix, wherein each identified second data item represents the same characteristic of each respective entity, although the value of each identified second data item may vary;
   selecting a row or column in the displayed c x d matrix;
   sorting the order of arrangement of the entities in the n x m matrix based on a comparison of the values of the identified second data items in the row or column of the n x m matrix corresponding to the row or column selected from the displayed c x d matrix;
   reordering the order of arrangement of the entities in the n x m matrix based on the sort performed on the second data items; and
   displaying the first c x d graphical representations of the identified data items from the reordered n x m matrix for observation by a user, for visual identification of changes in the relationships between the graphical representations.

7. The method of claim 1, further comprising the steps of:

   selecting a displayed item e in a selected column or a displayed item f in a selected row wherein item e is displayed in a row other than the first row of the selected column and the item f is displayed in a column other than the first column of the selected row;
   sub-sorting the n x m matrix by rank ordering the identified items e through m or f through n in the selected column or row and reordering items e through m or f through n according to rank, wherein upon reordering items e through m or f through n, the rows in which the respective items e through m are located, or columns in which the respective items f through n are reordered to the respective rank positions in the n x m matrix.; and
   displaying the first c x d graphical representations of the identified data items from the reordered n x m matrix for observation by a user, for visual identification of changes in the relationships between the graphical representations.

8. The method of claim 1, further comprising the steps of

   selecting a row g other than the first row of the reordered, displayed c x d matrix, or selecting a column h other than the first column of the reordered, displayed c x d matrix;

sub-sorting rows g through m or columns h through n by similarity sorting the rows of the n x m matrix from row g to row m or columns of the n x m matrix from columns h to column n, wherein the selected row is maintained as row position g or selected column is maintained as column position h in the reordered n x m matrix and all rows below row g are processed to determine a relative similarity value to selected row g, or all columns from h+1 to n are processed to determine a relative similarity value to selected column h, and wherein upon reordering, all other rows below row g are repositioned in descending order from row g, based on ranking by the relative similarity values to row g, or columns g +1 to n are repositioned in descending order from column h, based on ranking by the relative similarity values to column h, and wherein the first d rows or first c columns of the reordered m x n matrix are then displayed.

9. The method of claim 1, further comprising the steps of

selecting a row g or column h other than the first row or column of the reordered, displayed c x d matrix;
sub-sorting rows g through m or columns h through n of the n x m matrix from row g to row m or column h to column n, wherein the row g is maintained as row position g in the reordered n x m matrix and rows g + 1 to m are processed to determine a relative similarity to row g or column h is maintained as column position h in the reordered n x m matrix, the row having the nearest relative similarity value to row g is reordered as row g + 1 or the column having the nearest relative similarity value to column h is reordered as column h + 1 of the reordered n x m matrix, all remaining rows or columns which have not been reordered are then processed to determine a relative similarity value to reordered row g + 1 or reordered column h + 1,, the row having the nearest relative similarity to row g + 1 is reordered as row g + 2 or the column having the nearest relative similarity to column h +1 is reordered as column h + 2, and this process is reiterated until all rows g to m or columns h to n have been sub-sorted by nearest neighbor, wherein the first d rows or c columns of the reordered m x n matrix are then displayed.

10. The method of claim 1, further comprising manually repositioning at least one column or row of the displayed c x d matrix, wherein such manually repositioning repositions the corresponding rows and columns of the entire n x m matrix.

11. The method of claim 1, further comprising the steps of :

inputting (S3 or S7) additional data into the memory of the processing system, wherein the additional data corresponds to the entities in the n x m matrix and is arranged in an n x y matrix or a z x m matrix;
linking (S5 or S9) the column addresses (n values) of the n x y matrix or row addresses (m values) of the z x m matrix with those of the n x m matrix; and
displaying (130 or 120) the data from the n x y matrix or z x m matrix together with the first c x (d - y) graphical representations or (c-z) x d graphical representations (110) of the identified data items in a c x d matrix;

wherein, upon selecting a row or column in the displayed c x d matrix for sorting, only data items from the n x m matrix are considered for sorting procedures, and wherein, when a column of data is reordered in the n x m matrix according to the results of a sorting procedure, a corresponding column in the n x y matrix is reordered by its linking address to maintain the same relative display position to the reordered column of the n x m matrix when displayed in the c x d matrix on the display, and wherein, when a row of data is reordered in the n x m matrix according to the results of a sorting procedure, a corresponding row in the z x m matrix is reordered by its linking address to maintain the same relative display position to the reordered row of the n x m matrix when displayed in the c x d matrix on the display.

12. The method of claim 11, further comprising the steps of :

inputting further additional data (S3 or S7) into the memory of the processing system, wherein the further additional data corresponds to the entities in the n x m matrix and is arranged in a z x m matrix or in an n x y matrix;
linking (S5 or S9) the row addresses (m values) of the z x m matrix or the column addresses (n values) of the n x y matrix with those of the n x m matrix; and
displaying (120 or 130) the data from the z x m matrix or n x y matrix together with data from the n x y matrix and the first (c - z) x (d - y) graphical representations of the identified data items in a c x d matrix;

wherein, upon selecting a row or column in the displayed c x d matrix for sorting, only data items from the n x m matrix are considered for sorting procedures, and wherein, when a row of data is reordered in the n x m matrix according to the results of a sorting procedure, a corresponding row in the z x m matrix is reordered by its linking address to maintain the same relative display position to the reordered row of the n x m matrix when displayed in the c x d matrix on the display, and wherein, when a column of data is reordered in the n x m matrix according to the results of a sorting procedure, a corresponding column in the n x y m matrix is reordered by its linking address to maintain the same relative display position to the reordered column of the n x m matrix when displayed in the c x d matrix on the display.

13. The method of claim 1, wherein the entities are derived from microarray experiments, the identified data items are gene expression ratios and the graphical representations of the identified data items are color-coded graphical representations typically used in heat maps.

14. A data display system comprising a processor, a display and an input device, the processor being arranged to accept a plurality of data values comprising an n x m matrix, reorder the data values to group associated data values, encode the data values and output selected encoded data values to the display in the form of a heat map, wherein the processor is arranged to output as much of a group of data values corresponding to a selected data value as possible in dependence on the display size, the processor being arranged to accept inputs via the input device to manipulate the displayed heat map to display different groups.

15. A data display system according to claim 14, further comprising a database including data on said data values, the processor being arranged to accept a user input with respect to a displayed encoded data value, to obtain data from the database on said data value and to output said data to the display.

16. A data display system according to claim 14 or 15, wherein the processor is arranged to accept a number of different pluralities of data values, each plurality of data values being displayed on said display in a viewer, the viewers for data values having same or similar column or row headers being joinable to provide a combined set of data values.

17. A computer program comprising computer program code means for performing all of the steps of any of claims 1 to 13.

18. A computer program as claimed in claim 17 embodied on a computer readable medium.

FIG. 1

FIG. 2

300

302

304

306

308

Relatively
low invasive
ability

No data
available for
patient

Identified
Patient
Clusters

FIG. 3

FIG. 4

Sorted on this column,
Sorting ONLY the rows
containing expression data

FIG. 5

FIG. 6

FIG. 7A

S13 — Display k x j matrix as a single visualization

S15 — Column sort?
- Y → S17 — Select a$^{th}$ column
- N → S25 — Row sort?

S17 — Select a$^{th}$ column

S19 — Sort cells 1,a through n,a according to sorting schema

S21 — Assign new column order to n x y matrix

S23 — Rearrange the columns of the n x m matrix and n x y matrix synchronously

S25 — Row sort?
- Y → S27 — Select b$^{th}$ column
- N → S35 — Manual rearrangement?

S27 — Select b$^{th}$ column

S29 — Sort cells b,1 through b,m according to sorting schema

S31 — Assign new row order to z x m matrix

S33 — Rearrange the rows of the n x m matrix and n x y matrix synchronously

S35 — Manual rearrangement?
- N → End?
- Y → S37 — Drag and drop

S37 — Drag and drop

End?
- N
- Y → END

FIG. 7B

|  | 101 | 102 | 103 |
|---|---|---|---|
| 201 | 101,201 | 102,201 | 103,201 |
| 202 | 101,202 | 102,202 | 103,202 |
| 203 | 101,203 | 102,203 | 103,203 |
| 204 | 101,204 | 102,204 | 103,204 |

FIG. 8A

FIG. 8B

|   | 101 | 102 | 103 |
|---|---|---|---|
| 202 | 101,202 | 102,202 | 103,202 |
| 201 | 101,201 | 102,201 | 103,201 |
| 203 | 101,203 | 102,203 | 103,203 |
| 204 | 101,203 | 102,203 | 103,203 |

FIG. 8C

| | 101 | 102 | 103 |
|---|---|---|---|
| 202 | 101,202 | 102,202 | 103,202 |
| 203 | 101,203 | 102,203 | 103,203 |
| 201 | 101,201 | 102,201 | 103,201 |
| 204 | 101,204 | 102,204 | 103,204 |

FIG. 8D

FIG. 9

FIG. 10

When diameter reaches cell height or width, cell "fills"

FIG. 11

FIG. 12

FIG. 13

FIG. 14

FIG. 15

With common columns, the two windows are dockable

FIG. 16

EP 1 388 801 A2

FIG. 17

EP 1 388 801 A2

FIG. 18